# EUROPÄISCHE PATENTANMELDUNG

(11) **EP 2 072 502 A1**
(43) Veröffentlichungstag der Anmeldung: **24.06.2009**
(21) Anmeldenummer: 07076115.0
(22) Anmeldetag: 20.12.2007
(51) Int. Cl.: C07D 215/42, C07D 239/94, C07D 215/36, A61K 31/4706, A61K 31/517, A61P 35/00, A61P 25/28

(54) **Sulfoximid-substituierte Chinolin- und Chinazolinderivate als Kinase-Inhibitoren**

(71) Anmelder: Bayer Schering Pharma Aktiengesellschaft, 13353 Berlin (DE)
(72) Erfinder: Die Erfindernennung liegt noch nicht vor

(57) **Zusammenfassung**

Die vorliegende Erfindung betrifft Chinolin- bzw. Chinazolinderivate der allgemeinen Formel (I) : 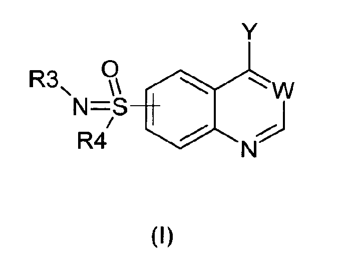 in der W, Y, R³ und R⁴ in der Beschreibung und den Ansprüchen angegeben sind, die Verwendung der Verbindungen der allgemeinen Formel (I) zur Behandlung verschiedener Erkrankungen, die Herstellung von Verbindungen der allgemeinen Formel (I), sowie Zwischenprodukte die in der Herstellung von Verbindungen der allgemeinen Formel (I) nützlich sind.

## Beschreibung

Die vorliegende Erfindung betrifft bestimmte Chinolin- bzw. Chinazolinderivate, deren Herstellung und Verwendung als Inhibitor von Proteinkinasen, insbesondere von Eph (*e*rythropoetin-*p*roducing *h*epatoma amplified sequence) - Rezeptoren zur Behandlung verschiedener Erkrankungen.

Proteintyrosinkinasen katalysieren die Phosphorylierung spezifischer Tyrosinreste in verschiedenen Proteinen. Solche Phosphorylierungsreaktionen spielen bei einer Vielzahl von zellulären Prozessen eine Rolle, die in der Regulation des Wachstums und der Differenzierung von Zellen involviert sind. Proteintyrosinkinasen werden in Rezeptor- und Nicht-Rezeptortyrosinkinasen eingeteilt. Die Familie der Rezeptortyrosinkinasen (RTKs) besteht aus 58 Kinasen (Manning G. et al. 2002, Science 298, 1912-1934). RTKs besitzen eine extrazelluläre Ligandenbindungsdomäne, eine Transmembrandomäne und eine intrazelluläre Domäne, die in der Regel die Tyrosinkinaseaktivität enthält. RTKs vermitteln die Signalweiterleitung von extrazellulären Stimulatoren wie z.B. Wachstumsfaktoren. Die Ligandenbindung führt zur Dimerisierung der RTKs und der wechselseitigen Autophosphorylierung ihrer intrazellulären Domänen. In Abhängigkeit vom Zelltyp werden dadurch spezifische intrazelluläre Bindungsproteine rekrutiert (u.a. Nicht-Rezeptortyrosinkinasen), über die eine Signalverarbeitung in der Zelle erfolgt (Schlessinger J. 2000, Cell 103, 211-225). Zu diesen zählen Rezeptorfamilien von Wachstumsfaktoren wie EGF (epidermal growth factor), VEGF (vascular endothelial growth factor), FGF (fibroblast growth factor), PDGF (platelet derived growth factor) und NGF (nerve growth factor), sowie der Insulinrezeptoren und die große Familie der Ephrinrezeptoren und andere.

Die größte Familie innerhalb der RTKs nehmen die Ephrin (Eph)-Rezeptoren ein. Sie teilen sich entsprechend ihrer sequenziellen Verwandtschaft und ihrer Ligandenspezifität in die Gruppe der EphA-Rezeptoren (9 Mitglieder) und der EphB-Rezeptoren (6 Mitglieder) auf (Kullander K. and Klein R. 2002, Nat.Rev.Mol.Cell Biol. 3, 475-486; Cheng N. et al. 2002, Cyt. and growth factor Rev. 13, 75-85.). Eph-Rezeptoren werden von membranständigen Liganden der EphrinA- bzw. EphrinB-Familie aktiviert. EphrinAs sind über Glykolipide (GPI) in der Zellmembran verankert, während EphrinBs eine Transmembranregion und eine intrazelluläre Domäne besitzen. Die Interaktion zwischen Ephrinen und den Eph-Rezeptoren führt zu einer bidirektionellen Signalübertragung in den ephrinexprimierenden und in den Eph-Rezeptor tragenden Zellen. Ephrine und Eph-Rezeptoren spielen in einer Vielzahl von morphogenetischen Prozessen in der Embryonalentwicklung und im adulten Organismus eine Rolle. Sie sind involviert in der embryonalen Musterbildung, in der Entwicklung des Blutgefäßsystems (Gerety S.S: et al 1999, Mol. Cell 4, 403-414) und in der Etablierung von neuronalen Verschaltungen (Flanagan, J.G. and Vanderhaeghen, P., 1998, Annu.Rev.Neurosci. 21, 306-354). Im adulten Organismus sind sie bei Neovascularisierungsprozesses z.B. bei der Tumorentstehung und bei der Endometriose, sowie bei der Morphogenese des Darmepithels beteiligt (Batlle E. et al. 2002, Cell 111:251-63.). Auf zellulärer Ebene vermitteln sie Migration, Adhäsion und juxtacrine Zellkontakte. Erhöhte Expression von Eph-Rezeptoren, wie z.B. EphB2 und EphB4 wurde auch in verschiedenen Tumorgeweben, wie z.B. Brust- und Darmtumoren beobachtet (Nakamoto M. and Bergemann A.D. 2002, Mic.Res.Tech. 59, 58-67). Knockout Mäuse von EphB2, EphB3 und EphB4 zeigen Defekte bei der Ausbildung des Blutgefäßsystems. Die embryonale Lethalität der EphB4 -/- Mäuse im Embryonalstadium d14, zeigt die besondere Rolle von EphB4 in diesem Prozeß (Gerety S.S: et al 1999, Mol. Cell 4, 403-414). Eine Modulation dieser Rezeptoren z.B. durch die Inhibition ihrer Kinaseaktivität führt beispielsweise dazu, dass das Tumorwachstum und/oder die Tumormetastasierung entweder durch eine direkte antitumorale oder durch eine indirekte antiangiogene Wirkung unterbunden wird.

Nicht-Rezeptortyrosinkinasen kommen in löslicher Form intrazellulär vor und sind an der Verarbeitung von extrazellulären Signalen (z.B. von Wachstumsfaktoren, Cytokinen, Antikörpern, Adhäsionsmolekülen) innerhalb der Zelle beteiligt. Zu ihnen zählen u.a. die Familien der src(sarcoma)-Kinasen, der Tec(tyrosine kinase expressed in hepatocellular carcinoma)-Kinasen, der Abl(Abelson)-kinasen und der Brk(breast tumor kinase)-Kinasen, sowie die fokale Adhesionskinase (FAK).

Eine veränderte Aktivität dieser Proteintyrosinkinasen kann zu verschiedensten physiologischen Störungen im menschlichen Organismus führen und dadurch z.B. entzündliche, neurologische und onkologische Erkankungen verursachen.

In WO 01/19828 A werden verschiedenste Kinaseinhibitoren offenbart.

In US 2004116388 A werden Triazinverbindungen offenbart, die Rezeptortyrosinkinasen inhibieren.

In WO 03/089434 A werden Imidazo[1,2a]pyrazin-8-yl-amine und in WO 04/00820 A verschiedene aromatische Monozyklen offenbart, die Rezeptortyrosinkinasen inhibieren.

In EP 0 187 705 A2 werden Imidazo[4,5f]-chinoline beschrieben, welche eine immunmodulierende Wirkung bei Infektionskrankheiten aufweisen. Ebenso beschreibt US 5,506,235 A Imidazo[4,5f]-chinoline mit immunstimulierender Wirkung.

In WO 04/006846 A werden verschiedene Chinazolinderivate offenbart, die Rezeptortryrosinkinasen inhibieren.

In WO 03/053960 werden substituierte 3-Cyanochinolin-Derivate als MEK-Inhibitoren beschrieben.

In US2005/0026933 werden Chinolin-Carbonitrile als ERFG-Inhibitoren beansprucht.

In WO 01/68186 werden Cyanochinoline zur Behandlung von Darm-Polypen beschrieben.

Unter den Rezeptortyrosinkinaseinhibitoren sind jedoch keine Eph-Rezeptorinhibitoren beschrieben.

Es ist Aufgabe der vorliegenden Erfindung, Verbindungen bereitzustellen, die Rezeptortyrosinkinasen, insbesondere Eph-Rezeptoren, inhibieren.

Die Aufgabe wird gelöst durch Chinolin- bzw. Chinazolinderivate mit der allgemeinen Formel (I), ein Verfahren zur Herstellung des Chinolin- bzw. Chinazolinderivats, die Verwendungen des Chinolin- bzw. Chinazolinderivats, sowie ein das Chinolin- bzw. Chinazolinderivat enthaltendes Arzneimittel, nach den folgenden Beschriebung und den Ansprüchen.

Die vorliegende Erfindung betrifft Chinolin- bzw. Chinazolinderivate mit der allgemeinen Formel (I) : 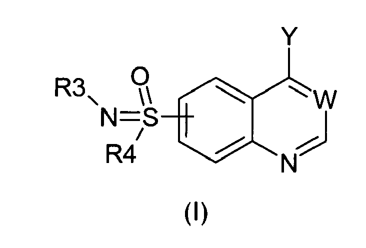 in der :
- W: gleich CH oder N ist ;
- Y: gleich NR¹R² oder OR¹ ist ;
- R¹ und R²: gleich oder verschieden und einfach oder mehrfach unabhängig voneinander ausgewählt sind aus der Gruppe, umfassend Wasserstoff, -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, -C₁-C₆-Alkyl-C₁-C₆-alkoxy, -C₁-C₆-Alkyl-C₁-C₆-alkoxy-C₁-C₆-alkoxy, -(CH₂)ₙ-C₆-C₁₂-Aryl, -(CH₂)ₙ-C₅-C₁₈-Heteroaryl,-(CH₂)ₙ-C₃-C₁₀-Cycloalkyl, -(CH₂)ₙ-C₃-C₁₂-Heterocycloalkyl, -Phenylen-(CH₂)p-R⁶, -(CH₂)ₚPO₃(R⁶)₂, -(CH₂)ₚ-NR⁴C(=S)R⁵, -(CH₂)ₚ-NR⁴S(=O)R⁵, -(CH₂)ₚ-NR⁴C(=O)NR⁵R⁶ -(CH₂)ₚ-NR⁴C(=O)OR⁵ -(CH₂)ₚ-NR⁴C(=NH)NR⁵R⁶, -(CH₂)ₚ-NR⁴C(=S)NR⁵R⁶, -(CH₂)ₚ-NR⁴S(=O)NR⁵R⁶, -(CH₂)ₚ-NR⁴S(=O)₂NR⁵R⁶, -(CH₂)ₚ-C(=O)R⁵, -(CH₂)ₚ-C(=S)R⁵, -(CH₂)ₚ-S(=O)R⁵, -(CH₂)ₚ-S(O)(NH)R^{5,} -(CH₂)ₚ-S(O)₂R⁵, -(CH₂)ₚ-SO₂OR⁵, -(CH₂)ₚ-CO₂R⁵, -(CH₂)ₚ-CSNR⁵R^{6,} -CHR⁵R⁶ und -(CH₂)ₚ-SR⁵ wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl oder -C₁-C₆-Alkoxy unsubstituiert sind oder einfach oder mehrfach unabhängig voneinander mit Hydroxy, Halogen, Nitro, Cyano, -NR⁵R⁶, -C(O)NR⁵R⁶, -S(O)₂NR⁵R⁶, -NR⁵S(O)₂R⁶, -NR⁵C(O)R⁶, -SR⁵ , -R⁵, oder -OR⁵ substituiert sind, wobei das Kohlenstoffgerüst des -C₃-C₁₀-Cycloalkyls und des -C₁-C₁₀-Alkyls einfach oder mehrfachunabhängig voneinander Stickstoff-, Sauerstoff-, Schwefelatome, -NR⁴ oder C=0-Gruppen oder eine oder mehrere Doppelbindungen enthalten kann, oder R¹ und R² optional miteinander eine Brücke aus 3-10 Methyleneinheiten bilden, wobei bis zu zwei Methyleneinheiten mit 0, S oder -NR⁴ optional ersetzt werden, und wobei der Phenyl-Rest optional einfach oder mehrfach unabhängig voneinander mit Hydroxy, Halogen, Nitro, Cyano, Phenyl, -NR⁵R⁶, Alkyl oder -OR⁵ substituiert ist ;
worin wenn Y NR¹R² ist, und R² Wasserstoff ist, dann R¹ nicht Wasserstoff ist ;
und worin wenn Y OR¹ ist, dann R¹ nicht Wasserstoff ist ;
- R³: ausgewählt ist aus der Gruppe umfassend Wasserstoff, -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)NR⁵R⁶, C(=O)OR⁵, -NR⁵R⁶, Alkyl, -SR⁵ oder -OR⁵ substituiert ist,
- R⁴: ausgewählt ist aus der Gruppe umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)OR⁵, C(=O)NR⁵R⁶, -NR⁵R⁶, Alkyl, -SR⁵ oder -OR⁵ substituiert ist,
oder
- R³ und R⁴: können, über das jeweilige Stickstoffatom und das jeweilige Schwefelatom, an das sie angeknüpft sind, einen Ring bilden, mit der Ringgröße von 5- bis zu 10- Ringatomen, wahlweise bestehend aus den Atomen Kohlenstoff, Stickstoff, Sauerstoff oder Schwefel ;
- R⁵ und R⁶: gleich oder verschieden und unabhängig voneinander ausgewählt sind aus der Gruppe, umfassend Wasserstoff, -C₁-C₁₀-Alkyl, -C₂-C₁₀₋Alkenyl, -C₂-C₁₀-Alkinyl, -C₁-C₆-Alkoxy, -C₃-C₁₀-Cycloalkyl, -C₃-C₁₂-Heterocycloalkyl, -C₆-C₁₂-Aryl und -C₅-C₁₈-Heteroaryl, wobei -C₁-C₁₀-Alkyl, -C₂-C₁₀-Alkenyl, -C₂-C₁₀-Alkinyl, -C₁-C₆-Alkoxy, -C3-C₁₀-Cycloalkyl, -C3-C₁₂-Heterocycloalkyl, -C₆-C₁₂-Aryl oder -C₅-C₁₈-Heteroaryl unsubstituiert sind oder einfach oder mehrfach unabhängig voneinander mit Hydroxy, Halogen, Cyano, Nitro, -OR⁷, -NR⁷R⁸, -C(O)NR⁷R⁸, -C(O)OR⁷ oder -C₁-C₆-Alkyl, wobei -C₁-C₆-Alkyl unsubstituiert ist oder einfach oder mehrfach unabhängig voneinander mit Halogen, Hydroxy, Cyano, -NR⁷R⁸, -OR⁷ oder Phenyl; oder R⁵ und R⁶ optional miteinander eine Brücke aus 3-10 Methyleneinheiten bilden, wobei bis zu zwei Methyleneinheiten mit 0, S oder NR⁴ optional ersetzt sein können;
- R⁷, R⁸: gleich oder verschieden und unabhängig voneinander ausgewählt sind aus der Gruppe, umfassend Wasserstoff, -C₁-C₄-Alkyl, - C₅-C₁₈-Aryl und - C₅-C₁₈-Heteroaryl, wobei -C₁-C₄-Alkyl, -C₆-C₁₂-Aryl, -C₅-C₁₈-Heteroaryl unsubstituiert ist oder einfach oder mehrfach unabhängig voneinander mit Halogen oder Alkoxy, oder R⁷ und R⁸ optional miteinander eine Brücke aus 3-10 Methyleneinheiten bilden, wobei bis zu zwei Methyleneinheiten mit 0, S oder -NR⁴ optional ersetzt sein können;
- n: = 1, 2, 3, 4, 5, oder 6,
- p: = 0, 1, 2, 3, 4, 5, oder 6, sowie
deren N-Oxide, Solvate, Hydrate, Stereoisomere, Diastereomere, Enantiomere und Salze.

Die Gruppen -C₁-C₆-Alkyl, -C₁-C₁₀-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl oder -C₁-C₆-Alkoxy, können einfach oder mehrfach, in einem Aspekt der Erfindung einfach, zweifach oder dreifach oder maximal so oft substituiert sein, wie sie Kohlenstoffatome haben, insbesondere einfach oder zweifach.

Eine bevorzugte Untergruppe sind Verbindungen, in denen :W gleich CH oder N ist ;
- Y: gleich NR¹R² oder OR¹ ist ;
- R¹: einfach oder mehrfach unabhängig voneinander ausgewählt ist aus der Gruppe, umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, -C₁-C₆-Alkyl-C₁-C₆-alkoxy, -C₁-C₆-Alkyl-C₁-C₆-alkoxy-C₁-C₆-alkoxy, -(CH₂)ₙ-C6-C₁₂-Aryl, -(CH₂)ₙ-C₅-C₁₈-Heteroaryl, -(CH₂)ₙ-C₃-C₁₀-Cycloalkyl, -(CH₂)ₙ-C₃-C₁₂-Heterocycloalkyl, -Phenylen-(CH₂)ₚ-R⁶, -(CH₂)ₚPO₃(R⁶)₂, -(CH₂)ₚ-NR⁴C(=S)R⁵,-(CH₂)ₚ-NR⁴S(=O)R⁵, -(CH₂)ₚ-NR⁴C(=O)NR⁵R⁶, -(CH₂)ₚ-NR⁴C(=O)OR⁵ -(CH₂)ₚ-NR⁴C(=NH)NR⁵R⁶, -(CH₂)ₚ-NR⁴C(=S)NR⁵R⁶, -(CH₂)ₚ-NR⁴S(=O)NR⁵R⁶, -(CH₂)ₚ- NR⁴S(=O)₂NR⁵R⁶, -(CH₂)ₚ-C(=O)R⁵, -(CH₂)ₚ-C(=S)R⁵, -(CH₂)ₚ-S(=O)R⁵, -(CH₂)ₚ-S(O)(NH)R⁵, -(CH₂)ₚ-S(O)₂R⁵, -(CH₂)ₚ-SO₂OR⁵, -(CH₂)ₚ-CO₂R⁵, -(CH₂)ₚ-CSNR⁵R⁶, -CHR⁵R⁶ und -(CH₂)ₚ-SR⁵; wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl oder -C₁-C₆-Alkoxy unsubstituiert ist oder einfach oder mehrfach unabhängig voneinander mit Hydroxy, Halogen, Nitro, Cyano, -NR⁵R⁶, -C(O)NR⁵R⁶, -S(O)₂NR⁵R⁶, -NR⁵S(O)₂R⁶, -NR⁵C(O)R⁶, -SR⁵, -R⁵, oder -OR⁵ substituiert ist, wobei das Kohlenstoffgerüst des -C₃-C₁₀-Cycloalkyls und des -C₁-C₁₀-Alkyls einfach oder mehrfach unabhängig voneinander Stickstoff-, Sauerstoff-, Schwefelatome, -NR⁴ oder C=O-Gruppen oder eine oder mehrere Doppelbindungen enthalten kann,
- R²: einfach oder mehrfach unabhängig voneinander ausgewählt ist aus der Gruppe, umfassend Wasserstoff, -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -(CH₂)ₙ-C₃-C₁₀-Cycloalkyl, -(CH₂)ₙ-C₃-C₁₂-Heterocycloalkyl, und -Phenylen-(CH₂)ₚ-R⁶, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl oder -C₅-C₁₈-Heteroaryl unsubstituiert ist, wobei das Kohlenstoffgerüst des -C₃-C₁₀-Cycloalkyls und des -C₁-C₁₀Alkyls einfach oder mehrfach unabhängig voneinander Stickstoff-, Sauerstoff-, Schwefelatome, -NR⁴ oder C=O-Gruppen oder eine oder mehrere Doppelbindungen enthalten kann ;
oder :
- R¹ und R²: optional miteinander eine Brücke aus 3-10 Methyleneinheiten bilden, wobei bis zu zwei Methyleneinheiten mit 0, S oder -NR⁴ optional ersetzt werden, und wobei der Phenyl-Rest optional einfach oder mehrfach unabhängig voneinander mit Hydroxy, Halogen, Nitro, Cyano, Phenyl, -NR⁵R⁶, Alkyl oder -OR⁵ substituiert ist ;
- R³: ausgewählt ist aus der Gruppe umfassend Wasserstoff, -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)NR⁵R⁶, C(=O)OR⁵, -NR⁵R⁶, Alkyl, -SR⁵ oder -OR⁵ substituiert ist,
- R⁴: ausgewählt ist aus der Gruppe umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)OR⁵, C(=O)NR⁵R⁶ , -NR⁵R⁶, Alkyl, -SR⁵ oder -OR⁵ substituiert ist,
- R⁵ und R⁶: gleich oder verschieden und unabhängig voneinander ausgewählt sind aus der Gruppe, umfassend Wasserstoff, -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -C₆-C₁₂-Aryl und -C₅-C₁₈-Heteroaryl, wobei -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -C₆-C₁₂-Aryl oder -C₅-C₁₈-Heteroaryl unsubstituiert sind oder einfach oder mehrfach unabhängig voneinander mit Hydroxy, Halogen, Cyano, Nitro, -OR⁷, -NR⁷R⁸, -C(0)NR⁷R⁸, -C(O)OR⁷ oder -C₁-C₆-Alkyl, wobei -C₁-C₆-Alkyl unsubstituiert ist oder einfach oder mehrfach unabhängig voneinander mit Halogen, Hydroxy, Cyano, -NR⁷R⁸, -OR⁷ oder Phenyl; oder R⁵ und R⁶ optional miteinander eine Brücke aus 3-10 Methyleneinheiten bilden, wobei bis zu zwei Methyleneinheiten mit 0, S oder NR⁴ optional ersetzt sein können;
- R⁷, R⁸: gleich oder verschieden und unabhängig voneinander ausgewählt sind aus der Gruppe, umfassend Wasserstoff, -C₁-C₄-Alkyl, - C₅-C₁₈-Aryl und - C₅-C₁₈-Heteroaryl, wobei -C₁-C₄-Alkyl, -C₆-C₁₂-Aryl, -C₅-C₁₈-Heteroaryl unsubstituiert ist oder einfach oder mehrfach unabhängig voneinander mit Halogen oder Alkoxy, oder R⁷ und R⁸ optional miteinander eine Brücke aus 3-10 Methyleneinheiten bilden, wobei bis zu zwei Methyleneinheiten mit 0, S oder -NR⁴ optional ersetzt sein können;
- n: = 1, 2, 3, 4, 5, oder 6,
- p: = 0, 1, 2, 3, 4, 5, oder 6, sowie
deren N-Oxide, Solvate, Hydrate, Stereoisomere, Diastereomere, Enantiomere und Salze.

Eine besonders bevorzugte Untergruppe sind Verbindungen, in denen :
- W: gleich CH oder N ist ;
- Y: gleich NR¹R² oder OR¹ ist ;
- R¹: einfach oder mehrfach unabhängig voneinander ausgewählt ist aus der Gruppe, umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, -C₁-C₆-Alkyl-C₁-C₆-alkoxy, -C₁-C₆-Alkyl-C₁-C₆-alkoxy-C₁-C₆-alkoxy, -(CH₂)ₙ-C₆-C₁₂-Aryl, -(CH₂)ₙ-C₅-C₁₈-Heteroaryl, -(CH₂)ₙ-C₃-C₁₀-Cycloalkyl, -(CH₂)ₙ-C₃-C₁₂-Heterocycloalkyl, -Phenylen-(CH₂)ₚ-R⁶, -(CH₂)ₚPO₃(R⁶)₂, -(CH₂)ₚ-NR⁴C(=S)R^{5,} -(CH₂)ₚ-NR⁴S(=O)R⁵, -(CH₂)ₚ-NR⁴C(=O)NR⁵R⁶, -(CH₂)ₚ-NR⁴C(=O)OR⁵, -(CH₂)ₚ-NR⁴C(=NH)NR⁵R⁶, -(CH₂)ₚ-NR⁴C(=S)NR⁵R⁶, -(CH₂)ₚ-NR⁴S(=O)NR⁵R^{6,} -(CH₂)ₚ-NR⁴S(=O)₂NR⁵R⁶, -(CH₂)ₚ-C(=O)R⁵, -(CH₂)ₚ-C(=S)R⁵, -(CH₂)ₚ-S(=O)R⁵, -(CH₂)ₚ-S(O)(NH)R⁵, -(CH₂)ₚS(O)₂R⁵, -(CH₂)ₚ-SO₂OR⁵, -(CH₂)ₚ-CO₂R⁵, -(CH₂)ₚ-CSNR⁵R⁶, -CHR⁵R⁶ und -(CH₂)ₚ-SR⁵ ; wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl oder -C₁-C₆-Alkoxy unsubstituiert ist oder einfach oder mehrfach unabhängig voneinander mit Hydroxy, Halogen, Nitro, Cyano, -NR⁵R⁶, -C(O)NR⁵R⁶, -S(O)₂NR⁵R⁶, -NR⁵S(O)₂R⁶, -NR⁵C(O)R⁶, -SR⁵, -R⁵, oder -OR⁵ substituiert ist, wobei das Kohlenstoffgerüst des -C₃-C₁₀-Cycloalkyls und des -C₁-C₁₀-Alkyls einfach oder mehrfach unabhängig voneinander Stickstoff-, Sauerstoff-, Schwefelatome, -NR⁴ oder C=O-Gruppen oder eine oder mehrere Doppelbindungen enthalten kann,
- R²: einfach oder mehrfach unabhängig voneinander ausgewählt ist aus der Gruppe, umfassend Wasserstoff, oder -C₁-C₆-Alkyl ;
- R²: ausgewählt ist aus der Gruppe umfassend Wasserstoff, -C₁-C₆-Alkyl, -C₂-C₆-Alkenyl, -C₂-C₆-Alkinyl, -C₃-C₁₀Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)NR⁵R⁶, C(=O)OR⁵, -NR⁵R⁶, Alkyl, -SR⁵ oder -OR⁵ substituiert ist,
- R⁴: ausgewählt ist aus der Gruppe umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)OR⁵, C(=O)NR⁵R⁶, -NR⁵R⁶, Alkyl, -SR⁵ oder -OR⁵ substituiert ist,
- R⁵ und R⁶: gleich oder verschieden und unabhängig voneinander ausgewählt sind aus der Gruppe, umfassend Wasserstoff, -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -C₆-C₁₂-Aryl und -C₅-C₁₈-Heteroaryl, wobei -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -C₆-C₁₂-Aryl oder -C₅-C₁₈-Heteroaryl unsubstituiert sind oder einfach oder mehrfach unabhängig voneinander mit Hydroxy, Halogen, Cyano, Nitro, -OR⁷, -NR⁷R⁸, -C(O)NR⁷R⁸, -C(O)OR⁷ oder -C₁-C₆-Alkyl, wobei -C₁-C₆-Alkyl unsubstituiert ist oder einfach oder mehrfach unabhängig voneinander mit Halogen, Hydroxy, Cyano, -NR⁷R⁸, -OR⁷ oder Phenyl; oder R⁵ und R⁶ optional miteinander eine Brücke aus 3-10 Methyleneinheiten bilden, wobei bis zu zwei Methyleneinheiten mit 0, S oder NR⁴ optional ersetzt sein können;
- R⁷, R⁸: gleich oder verschieden und unabhängig voneinander ausgewählt sind aus der Gruppe, umfassend Wasserstoff, -C₁-C₄-Alkyl, - C₅-C₁₈-Aryl und - C₅-C₁₈-Heteroaryl, wobei -C₁-C₄-Alkyl, -C₆-C₁₂-Aryl, -C₅-C₁₈-Heteroaryl unsubstituiert ist oder einfach oder mehrfach unabhängig voneinander mit Halogen oder Alkoxy, oder R⁷ und R⁸ optional miteinander eine Brücke aus 3-10 Methyleneinheiten bilden, wobei bis zu zwei Methyleneinheiten mit 0, S oder -NR⁴ optional ersetzt sein können;
- n: = 1, 2, 3, 4, 5, oder 6,
- p: = 0, 1, 2, 3, 4, 5, oder 6, sowie
deren N-Oxide, Solvate, Hydrate, Stereoisomere, Diastereomere, Enantiomere und Salze.

Eine ganz besonders bevorzugte Untergruppe sind Verbindungen, in denen :
- W: gleich CH oder N ist ;
- Y: gleich NR¹R² oder OR¹ ist ;
- R¹: einfach oder mehrfach unabhängig voneinander ausgewählt ist aus der Gruppe, umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, -C₁-C₆-Alkyl-C₁-C₆-alkoxy, -C₁-C₆-Alkyl-C₁-C₆-alkoxy-C₁-C₆-alkoxy, -(CH₂)ₙ-C6-C₁₂-Aryl, -(CH₂)ₙ-C₅-C₁₈-Heteroaryl, -(CH₂)ₙ-C3-C₁₀-Cycloalkyl, -(CH₂)ₙ-C₃-C₁₂-Heterocycloalkyl, -Phenylen-(CH₂)ₚ-R⁶, -(CH₂)ₚPO₃(R⁶)₂, -(CH₂)ₚ-NR⁴C(=S)R⁵, -(CH₂)ₚ-NR⁴S(=O)R⁵, -(CH₂)ₚ-NR⁴C(=O)NR⁵R⁶, -(CH₂)p-NR⁴C(=O)OR⁵, -(CH₂)ₚ-NR⁴C(=NH)NR⁵R⁶, -(CH₂)ₚ₋NR⁴C(=S)NR⁵R⁶, -(CH₂)ₚ-NR⁴S(=O)NR⁵R⁶_{,} -(CH₂)ₚ-NR⁴S(=O)₂NR⁵R⁶, -(CH₂)ₚ-C(=O)R⁵, -(CH₂)ₚ-C(=S)R⁵, -(CH₂)ₚ-S(=O)R⁵, -(CH₂)ₚ-S(O)(NH)R⁵, -(CH₂)ₚ-S(O)₂R⁵, -(CH₂)ₚ-SO₂OR⁵, -(CH₂)ₚ-CO₂R⁵, -(CH₂)ₚ-CSNR⁵R⁶, -CHR⁵R⁶ und -(CH₂)ₚ-SR⁵ ; wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl oder -C₁-C₆-Alkoxy unsubstituiert ist oder einfach oder mehrfach unabhängig voneinander mit Hydroxy, Halogen, Nitro, Cyano, -NR⁵R⁶, -C(O)NR⁵R⁶, -S(O)₂NR⁵R⁶, -NR⁵S(O)₂R⁶, -NRC(O)R⁶, -SR⁵, -R⁵, oder -OR⁵ substituiert ist, wobei das Kohlenstoffgerüst des -C₃-C₁₀-Cycloalkyls und des -C₁-C₁₀-Alkyls einfach oder mehrfach unabhängig voneinander Stickstoff-, Sauerstoff-, Schwefelatome, -NR⁴ oder C=O-Gruppen oder eine oder mehrere Doppelbindungen enthalten kann,
- R²: einfach oder mehrfach unabhängig voneinander ausgewählt ist aus der Gruppe, umfassend Wasserstoff, oder -C₁-C₆-Alkyl ;
- R³: ausgewählt ist aus der Gruppe umfassend Wasserstoff, -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)NR⁵R⁶, C(=O)OR⁵, -NR⁵R⁶, Alkyl, -SR⁵ oder -OR⁵ substituiert ist,
- R⁴: ausgewählt ist aus der Gruppe umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, oder -C₅-C₁₈-Heteroaryl, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)OR⁵, C(=O)NR⁵R⁶, -NR⁵R⁶, Alkyl, -SR⁵ oder -OR⁵ substituiert ist,
- R⁵ und R⁶: gleich oder verschieden und unabhängig voneinander ausgewählt sind aus der Gruppe, umfassend Wasserstoff, -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -C₆-C₁₂-Aryl und -C₅-C₁₈-Heteroaryl, wobei -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -C₆-C₁₂-Aryl oder -C₅-C₁₈-Heteroaryl unsubstituiert sind oder einfach oder mehrfach unabhängig voneinander mit Hydroxy, Halogen, Cyano, Nitro, -OR⁷, -NR⁷R⁸, -C(O)NR⁷R⁸, -C(O)OR⁷ oder -C₁-C₆₋Alkyl, wobei -C₁-C₆-Alkyl unsubstituiert ist oder einfach oder mehrfach unabhängig voneinander mit Halogen, Hydroxy, Cyano, -NR⁷R⁸, -OR⁷ oder Phenyl; oder R⁵ und R⁶ optional miteinander eine Brücke aus 3-10 Methyleneinheiten bilden, wobei bis zu zwei Methyleneinheiten mit 0, S oder NR⁴ optional ersetzt sein können;
- R⁷, R⁸: gleich oder verschieden und unabhängig voneinander ausgewählt sind aus der Gruppe, umfassend Wasserstoff, -C₁-C₄-Alkyl, - C₅-C₁₈-Aryl und - C₅-C₁₈-Heteroaryl, wobei -C₁-C₄-Alkyl, -C₆-C₁₂-Aryl, -C₅-C₁₈-Heteroaryl unsubstituiert ist oder einfach oder mehrfach unabhängig voneinander mit Halogen oder Alkoxy, oder R⁷ und R⁸ optional miteinander eine Brücke aus 3-10 Methyleneinheiten bilden, wobei bis zu zwei Methyleneinheiten mit 0, S oder -NR⁴ optional ersetzt sein können;
- n: = 1, 2, 3, 4, 5, oder 6,
- p: = 0, 1, 2, 3, 4, 5, oder 6, sowie
deren N-Oxide, Solvate, Hydrate, Stereoisomere, Diastereomere, Enantiomere und Salze.

Noch bevorzugter sind folgende Verbindungen :
S-[4-(3-Hydroxyphenyl-1-yl)-aminochinolin-6-yl]-S-methylsulfoximid ;
S-[4-(3-Hydroxy-6-methyl-phenyl-1-yl)-aminochinolin-6-yl]-S-methylsulfoximid ;
S-[4-(3-Hydroxy-5-methoxy-phenyl-1-yl)-aminochinolin-6-yl]-S-methylsulfoximid ;
S-[4-(6-Chlor-3-hydroxy-phenyl-1-yl)-aminochinolin-6-yl]-S-methylsulfoximid ;
S-[4-(3-Hydroxy-5-methoxy-phenyl-1-yl)-hydroxychinolin-6-yl]-S-methylsulfoximid ;
N,S-Dimethyl-S-[4-(3-hydroxyphenyl-1-yl)-aminochinolin-6-yl]-sulfoximid ;
N,S-Dimethyl-S-[4-(3-hydroxy-6-methyl-phenyl-1-yl)-aminochinolin-6-yl]-sulfoximid ;
N, S-Dimethyl-S-[4-(3-hydroxy-5-methoxy-phenyl-1-yl)-aminochinolin-6-yl]-sulfoximid ;
N-Acetyl-S-[4-(3-hydroxy-5-methoxy-phenyl-1-yl)-aminochinolin-6-yl]-S-methylsulfoximid ;
N-Acetyl-S-[4-(3-Hydroxy-6-methyl-phenyl-1-yl)-aminochinolin-6-yl]-S-methylsulfoximid ;
S-[4-(3-Hydroxy-6-methyl-phenyl-1-yl)-aminochinolin-6-yl]-N-methoxycarbonyl-S-methylsulfoximid ;
S-[4-(3-Hydroxy-5-methoxy-phenyl-1-yl)-aminochinolin-6-yl]-N-methoxycarbonyl-S-methylsulfoximid ;
N-(Ethylamino)carbonyl-S-[4-(3-Hydroxy-5-methoxy-phenyl-1-yl)-aminochinolin-6-yl]-S-methylsulfoximid ;
N-(Ethylamino)carbonyl-S-[4-(3-Hydroxy-6-methyl-phenyl-1-yl)-aminochinolin-6-yl]-S-methylsulfoximid ;
S-[4-(3-Hydroxy-5-methoxy-phenyl-1-yl)-aminochinazolin-6-yl]-S-methylsulfoximid ;
S-[4-(3-Hydroxy-phenyl-1-yl)-aminochinazolin-6-yl]-S-methylsulfoximid ;
S-[4-(3-Hydroxy-6-methyl-phenyl-1-yl)-aminochinazotin-6-yl]-S-methylsulfoximid ;
S-[4-(3-Hydroxy-6-methyl-phenyl-1-yl)-aminochinazolin-6-yl]-S-methylsulfoximid ;

Es wurde gefunden, dass die erfindungsgemäßen Verbindungen Rezeptortyrosinkinasen, insbesondere Eph-Rezeptoren, inhibieren können.

Unter Alkyl ist jeweils ein geradkettiger oder verzweigter Alkylrest, wie beispielsweise Methyl, Ethyl, Propyl, Isopropyl, Butyl, Isobutyl, *sek*.-Butyl, *tert*-Butyl, Pentyl, Isopentyl, Hexyl, Heptyl, Octyl, Nonyl und Decyl, zu verstehen.

Unter Alkoxy ist jeweils ein geradkettiger oder verzweigter Alkoxyrest, wie beispielsweise Methyloxy, Ethyloxy, Propyloxy, Isopropyloxy, Butyloxy, Isobutyloxy, *sek.* Butyloxy, Pentyloxy, Isopentyloxy, Hexyloxy, Heptyloxy, Octyloxy, Nonyloxy oder Decyloxy zu verstehen.

Die Alkenyl-Substituenten sind jeweils geradkettig oder verzweigt, wobei beispielsweise folgenden Reste gemeint sind: Vinyl, Propen-1-yl, Propen-2-yl, But-1-en-1-yl, But-1-en-2-yl, But-2-en-1-yl, But-2-en-2-yl, 2-Methyl-prop-2-en-1-yl, 2-Methyl-prop-1-en-1-yl, But-1-en-3-yl, But-3-en-1-yl, Allyl.

Unter Alkinyl ist jeweils ein geradkettiger oder verzweigter Alkinyl-Rest zu verstehen, der zwei bis sechs, bevorzugt zwei bis vier C-Atome enthält. Beispielsweise sind die folgenden Reste geeignet: Ethinyl, Propin-1-yl, Propin-3-yl, But-1-in-1-yl, But-1-in-4-yl, But-2-in-1-yl, But-1-in-3-yl.

Unter Cycloalkyl sind monocyclische Alkylringe wie Cyclopropyl, Cyclobutyl, Cyclopentyl, Cyclohexyl oder Cycloheptyl, aber auch bicyclische Ringe oder tricyclische Ringe wie zum Beispiel Adamantanyl zu verstehen. Die Cycloalkylringe können unsubstituiert oder ein- oder mehrfach substituiert sein. Cycloalkyle gemäß dieser Erfindung enthalten C₃-C₁₂ Kohlenwasserstoffatomen, bevorzugt sind Cycloalkyle mit C₃-C₁₀-Kohlenwasserstoffatomen und besonders bevorzugt sind Cycloalkyle mit C₃-C₆-Kohlenwasserstoffatomen.

Der Heteroarylrest umfaßt ein aromatisches Ringsystem, welches jeweils 5 - 18 Ringatome, bevorzugt 5 bis 10 Ringatome und besonders bevorzugt 5 bis 7 Ringatome enthält und anstelle des Kohlenstoffs ein oder mehrere gleiche oder verschiedene Heteroatome aus der Gruppe Sauerstoff, Stickstoff oder Schwefel. Der Rest kann mono-, bi- oder tricyclisch und zusätzlich jeweils benzokondensiert sein. Es sind allerdings nur diejenigen Kombinationen gemeint, die aus Sicht eines Fachmanns sinnvoll sind, insbesondere in Bezug auf die Ringspannung.

Die Heteroarylringe können unsubstituiert oder ein- oder mehrfach substituiert sein. Beispielhaft seien genannt: Thienyl, Furanyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, Pyridyl, Pyridazinyl, Pyrimidinyl, Pyrazinyl, Triazinyl sowie Benzoderivate dieser Reste, wie z. B. 1,3-Benzodioxolyl, Benzofuranyl, Benzothienyl, Benzoxazolyl, Benzimidazolyl, Indazolyl, Indolyl, Isoindolyl, Oxepinyl, Azocinyl, Indolizinyl, Indolyl, Isoindolyl, Indazolyl, Benzimidazolyl, Purinyl, Chinolinyl, Isochinolinyl, Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl etc.. Die Heteroarylringe können dabei über jedes Kohlenstoffatom des Ringes angeknüpft sein.

Unter Halogen ist jeweils Fluor, Chlor, Brom oder Jod zu verstehen.

C₃-C₁₂-Heterocycloalkyl steht für einen 3 - 12 Kohlenstoffatome, bevorzugt für einen 3 bis 10 Kohlenstoffatome und besonders bevorzugt für einen 3 bis 6 Kohlenstoffatome umfassenden Alkylring, der durch mindestens eins der folgenden Atome Stickstoff, Sauerstoff und/oder Schwefel im Ring unterbrochen ist und der gegebenenfalls durch ein oder mehrere, gleich oder verschiedene -(CO)-, -SO- oder -SO₂- Gruppen im Ring unterbrochen sein kann und gegebenenfalls ein oder mehrere Doppelbindungen im Ring enthält. Es sind allerdings nur diejenigen Kombinationen gemeint, die aus Sicht eines Fachmanns sinnvoll sind, insbesondere in Bezug auf die Ringspannung. C₃-C₁₂-Herterocycloalkyle gemäß dieser Erfindung sind monocyclisch, aber auch bicyclisch oder tricyclisch. Als monocyclische Heterocyclyle seien z. B. genannt: Oxiranyl, Oxethanyl, Aziridinyl, Azetidinyl, Tetrahydrofuranyl, Tetrahydrothiophenyl, Pyrrolidinyl, Dioxolanyl, Oxazolidinyl, Isoxazolidinyl, Thiazolidinyl, Imidazolidinyl, Pyrazolidinyl, Dioxanyl, Piperidinyl, Tetrahydropyranyl, Morpholinyl, Dithianyl, Thiomorpholinyl, Piperazinyl, Trithianyl, Chinuclidinyl, Azepanyl, Oxepanyl etc. Beispiele für bicyclische und polycyclische Ringsysteme sind 7-Oxa-bicyclo[2.2.1]heptanyl, 7-Aza-bicyclo[2.2.1]heptanyl, Octahydro-indolyl, Octahydro-iso-indolyl, Indolizidinyl, Octahydro-indazolyl, 1,4-Diaza-bicyclo[2.2.2]octanyl oder auch 3-Aza-bicyclo[3.2.1]octanyl etc.

Ein Arylrest hat jeweils 6 - 12 Kohlenstoffatome. Der Rest kann mono- oder bicyclisch, beispielsweise Naphthyl, Biphenyl und insbesondere Phenyl, sein.

So wie in dieser Anmeldung verwendet, bezeichnet "C₁-C₁₀", zum Beispiel im Zusammenhang mit der Definition von "C₁-C₁₀-Alkyl" eine Alkylgruppe mit einer endlichen Anzahl von 1 bis 10 Kohlenstoffatomen, d.h. 1, 2, 3, 4, 5, 6, 7, 8, 9 oder 10 Kohlenstoffatome. Weiter wird die Definition "C₁-C₁₀" so interpretiert, dass jeder mögliche Teilbereich, wie beispielsweise, C₁-C₁₀, C₂-C₉, C₃-C₈, C₄-C₇, C₅-C₆, C₁-C₂, C₁-C₃, C₁-C₄, C₁-C₅, C₁-C₆, C₁-C₇, C₁-C₈, C₁-C₉, C₁-C₁₀, bevorzugt C₁-C₂, C₁-C₃, C₁-C₄, C₁-C₅, C₁-C₆; bevorzugt C₁-C₄ in der Definition mitbeinhaltet ist.

Analog dazu, bezeichnet "C₂-C₁₀", zum Beispiel in Zusammenhang mit der Definition von "C₂-C₁₀-Alkenyl" und "C₂-C₁₀-Alkinyl" eine Alkenylgruppe oder Alkinylgruppe mit einer endlichen Anzahl von 2 bis 10 Kohlenstoffatomen, d.h. 2, 3, 4, 5, 6, 7, 8, 9 oder 10 Kohlenstoffatome. Die Definition "C₂-C₁₀" wird so interpretiert, dass jeder mögliche Teilbereich, wie beispielsweise C₂-C₁₀, C₃-C₉, C₄-C₈, C₅-C₇, C₂-C₃, C₂-C₄, C₂-C₅, C₂-C₆, C₂-C₇, C₂-C₈, C₂-C₉, bevorzugt C₂-C₄ in der Definition mitbeinhaltet ist.

Weiter bezeichnet "C₁-C₆", zum Beispiel in Zusammenhang mit der Definition von "C₁-C₆-Alkoxy" eine Alkoxygruppe mit einer endlichen Anzahl von 1 bis 6 Kohlenstoffatomen, d.h. 1, 2, 3, 4, 5 oder 6 Kohlenstoffatome. Die Definition "C₁-C₆" wird so interpretiert, dass jeder mögliche Teilbereich, wie beispielsweise C₁-C₆, C₂-C₅, C₃-C₄, C₁-C₂, C₁-C₃, C₁-C₄, C₁-C₅, C₁-C_{6 ;} bevorzugt C₁-C₄, in der Definition mitbeinhaltet ist.

Alle hier nicht explizit aufgeführten Bereichsangaben der Anmeldung sind analog wie die oben exemplarisch genannten Bereiche "C₁-C₁₀", "C₂-C₁₀" und "C₁-C₆" definiert.

Unter Isomeren sind chemische Verbindungen der gleichen Summenformel, aber unterschiedlicher chemischer Struktur, zu verstehen. Es werden im Allgemeinen Konstitutionsisomere und Stereoisomere unterschieden. Konstitutionsisomere besitzen die gleiche Summenformel, unterscheiden sich jedoch durch die Verknüpfungsweise ihrer Atome oder Atomgruppen. Hierzu zählen funktionelle Isomere, Stellungsisomere, Tautomere oder Valenzisomere. Stereoisomere haben grundsätzlich die gleiche Struktur (Konstitution) und damit auch die gleiche Summenformel, unterscheiden sich aber durch die räumliche Anordnung der Atome. Im Allgemeinen werden Konfigurationsisomere und Konformationsisomere unterschieden. Konfigurationsisomere sind Stereoisomere, die sich nur durch Bindungsbruch ineinander überführen lassen. Hierzu zählen Enantiomere, Diastereomere und E / Z (cis / trans) Isomere. Enantiomere sind Stereoisomere, die sich wie Bild und Spiegelbild zueinander verhalten und keine Symmetrieebene aufweisen, zum Beispiel, können die Verbindungen der Erfindung entweder als (R)- oder (S)-Sulfoximid existieren, oder als eine Mischung von (R)- und (S)-Sulfoximid. Alle Stereoisomere, die keine Enantiomere sind, bezeichnet man als Diastereomere. Ein Spezialfall sind E / Z (cis / trans) Isomere an Doppelbindungen. Konformationsisomere sind Stereoisomere, die sich durch die Drehung von Einfachbindungen ineinander überführen lassen. Zur Abgrenzung der Isomerie-Arten voneinander siehe auch die IUPAC Regeln Sektion E *(*Pure Appl. Chem. 1976, 45, 11-30).

Die erfindungsgemäßen Chinolin- bzw. Chinazolinderivate mit der allgemeinen Formel (I) beinhalten auch die möglichen tautomeren Formen und umfassen die E- oder Z-Isomeren oder, falls ein chirales Zentrum vorhanden ist, auch die Racemate und Enantiomeren. Hierunter sind auch Doppelbindungsisomeren zu verstehen.

Die erfindungsgemäßen Chinolin- bzw. Chinazolinderivate können auch in Form von Solvaten, insbesondere von Hydraten vorliegen, wobei die erfindungsgemäßen Verbindungen demgemäß polare Lösungsmittel, insbesondere von Wasser, als Strukturelement des Kristallgitters der erfindungsgemäßen Verbindungen enthalten. Der Anteil an polarem Lösungsmittel, insbesondere Wasser kann in einem stöchiometrischen oder auch unstöchiometrischen Verhältnis vorliegen. Bei stöchiometrischen Solvaten, Hydraten spricht man auch von Hemi-, (Semi-), Mono-, Sesqui-, Di-, Tri-, Tetra-, Penta-, usw. Solvaten oder Hydraten.

N-Oxide bedeutet, dass mindestens ein Stickstoff der erfindungsgemäßen Verbindungen der allgemeinen Formel (I) oxidiert sein kann.

Ist eine saure Funktion enthalten, sind als Salze die physiologisch verträglichen Salze organischer und anorganischer Basen geeignet, wie beispielsweise die gut löslichen Alkali- und Erdalkalisalze sowie Salze von N-Methyl-glukamin, Dimethyl-glukamin, Ethyl-glukamin, Lysin, 1,6-Hexadiamin, Ethanolamin, Glukosamin, Sarkosin, Serinol, Tris-hydroxy-methyl-aminomethan, Aminopropandiol, Sovak-Base, 1-Amino-2,3,4-butantriol.

Ist eine basische Funktion enthalten, sind die physiologisch verträglichen Salze organischer und anorganischer Säuren geeignet wie Salzsäure, Bromwasserstoffsäure, Schwefelsäure, Phosphorsäure, Oxalsäure, Malonsäure, Maleinsäure, Zitronensäure, Bernsteinsäure, Weinsäure u.a.

Funktionelle Gruppen können gegebenenfalls durch Schutzgruppen während der Reaktionsequenz geschützt werden. Solche Schutzgruppen können unter anderem Ester, Amide, Ketale/Acetale, Nitrogruppen, Carbamate, Alkylether, Allylether, Benzylether oder Silylether sein. Als Bestandteil von Silylethern können unter anderem Verbindungen, wie z.B. Trimethylsilyl (TMS), tert-Butyl-dimethylsilyl (TBDMS), tert-Butyl-diphenylsilyl (TBDPS), Triethylsilyl (TES), etc., vorkommen. Die Herstellung ausgewählter mit Schutzgruppen versehener Intermediate wird im experimentellen Teil beschrieben.

Die erfindungsgemäßen Chinolin- bzw. Chinazolinderivate mit der allgemeinen Formel (I) inhibieren Rezeptortyrosinkinasen, insbesondere Eph Kinasen, worauf auch deren Wirkung zum Beispiel bei der Behandlung von Erkrankungen, in denen die Angiogenese, Lymphangiogenese oder Vaskulogenese eine Rolle spielen, bei Erkrankungen der Blutgefäße, Erkrankungen, die durch eine Hyperproliferation von Körperzellen hervorgerufen werden oder chronischen oder akuten neurodegenerativen Erkrankungen. Die vorliegenden Chinolin- bzw. Chinazolinderivate mit der allgemeinen Formel (I) können demnach als Arzneimittel verwendet werden.

Behandlungen werden bevorzugt am Menschen, aber auch an verwandten Säugetierspezies wie z.B. Hund und Katze durchgeführt.

Angiogene und/oder vaskulogene Erkrankungen können behandelt werden, indem das Wachstum der Blutgefäße inhibiert (antiangiogen) oder gefördert (proangiogen) wird. Antiangiogene Verwendungen erfolgen z.B. bei der Tumorangiogenese, der Endometriose, bei diabetisch-bedingten oder anderen Retinopathien oder bei altersbedingter Degeneration der Makula. Proangiogene Verwendungen erfolgen z.B. bei Herzinfarkt oder akut neurodegenerativen Erkrankungen durch Ischämien des Gehirns oder Neurotraumata.

Unter Blutgefäßerkrankungen sind Stenosen, Arteriosklerosen, Restenosen oder Entzündungskrankheiten, wie rheumatische Arthritis, zu verstehen.

Unter hyperproliferativen Erkrankungen, sind solide Tumore, nicht-solide Tumore oder nicht-cancerogene Zellhyperproliferation in der Haut zu verstehen, wobei unter soliden Tumoren unter anderen Mamma-, Colon-, Nieren-, Lungen- und/oder Gehirntumore zu verstehen sind. Unter nichtsoliden Tumoren sind unter anderem Leukämien zu verstehen und unter nichtcancerogener Zellhyperproliferation in der Haut unter anderem Psoriasis, Ekzeme, Skleroderma oder benigne Hypertrophie der Prostata.

Unter chronisch neurodegenerativen Erkrankungen sind u.a. Huntington's Erkrankung, amyotrophe Lateralsklerose, Parkinsonsche Erkrankung, AIDS induzierte Dementia oder Alzheimer'sche Erkrankung zu verstehen.

Verwendung der Chinolin- bzw. Chinazolinderivate mit der allgemeinen Formel (I) können ebenfalls für diagnostische Zwecke *in vitro* oder *in vivo* zur Identifizierung von Rezeptoren in Geweben mittels Autoradioagraphie und/oder PET verwendet werden.

Insbesondere können die Substanzen für diagnostische Zwecke auch radiomarkiert sein.

Zur Verwendung der erfindungsgemäßen Chinolin- bzw. Chinazolinderivate als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer.

Diese pharmazeutischen Präparate sind ebenfalls Gegenstand der vorliegenden Erfindung.

Für die parenterale Anwendung sind insbesondere Injektionslösungen oder Suspensionen, insbesondere wässrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die enteralen, parenteralen und oralen Applikationen sind ebenfalls Gegenstand der vorliegenden Erfindung.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1.000 mg, wobei die Dosis als einmal zu verabreichende Einzeldosis oder unterteilt in zwei oder mehreren Tagesdosen gegeben werden kann.

Ebenfalls Gegenstand der vorliegenden Erfindung sind Arzneimittel zur Behandlung der oben aufgeführten Erkrankungen, die mindestens ein Chinolin bzw. Chinazolinderivat mit der allgemeinen Formel (I) enthalten, wobei die Arzneimittel gegebenenfalls geeignete Formulierungs- und Trägerstoffen enthalten können.

Soweit die Herstellung der Ausgangsverbindungen nicht beschrieben wird, sind diese dem Fachmann bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen.

Die Isomerengemische können nach üblichen Methoden wie beispielsweise Kristallisation, Chromatographie oder Salzbildung in die Enantiomeren bzw. E/Z-Isomeren aufgetrennt werden.

Die Herstellung der Salze erfolgt in üblicher Weise, indem man eine Lösung der Verbindung mit der allgemeinen Formel (I) mit der äquivalenten Menge oder einem Überschuss einer Base oder Säure, die gegebenenfalls in Lösung ist, versetzt und den Niederschlag abtrennt oder in üblicher Weise die Lösung aufarbeitet.

Ebenfalls Gegenstand der vorliegenden Erfindung ist das Verfahren zur Herstellung der erfindungsgemäßen Chinolin- bzw. Chinazolinderivate.

Die für die Herstellung der erfindungsgemäßen Chinolin- bzw. Chinazolinderivate mit der allgemeinen Formel (I) vorzugsweise verwendeten Zwischenprodukte sind folgende Verbindungen mit den allgemeinen Formeln (I) bis (V).

### Allgemeine Beschreibung der Herstellung der erfindungsgemäßen Verbindungen:

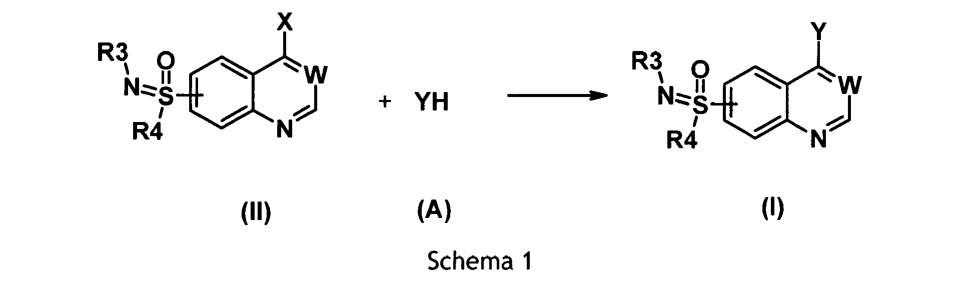

Erfindungsgemäße Chinolin- bzw. Chinazolin-derivate mit der allgemeinen Formel (I) können zum Beispiel auf dem im Schema 1 gezeigten Weg von den Zwischenprodukten (II) und (A) hergestellt werden, worin der Rest X beispielsweise Hal, OS(O)₂Aryl oder -OS(O)₂CₙF₂ₙ₊₁ mit n= 1-4, Hal für ein Halogen-Atom wie Fluorid, Chlorid, Bromid oder lodid und die Reste R³ und R⁴ wie in den Claims beschrieben sein kann und die Reste W und YH dieselbe Bedeutung haben wie in der allgemeinen Formel (I), mit H als Wasserstoff. Die benötigten Ausgangsmaterialien sind entweder kommerziell erhältlich oder werden nach in der Literatur bekannten Verfahren bzw. in Analogie zu in der Literatur bekannten Verfahren oder wie im Folgenden beschrieben hergestellt.

Durch Substitution einer geeigneten funktionellen Gruppe X mit einem Nucleophil wie ggf. mit Schutzgruppen versehenen primären oder sekundären Aminen oder Alkoholen an einer Verbindung mit der allgemeinen Formel (II) werden Verbindungen mit der allgemeinen Formel (I) gebildet (z.B. J. Med. Chem., 2005, 48, 3354-3363; J. Med. Chem., 1995, 38, 3482-3287). Für diese Umsetzung kann u.U. der Zusatz von Basen notwendig sein *(*J. Med. Chem., 2001, 44, 3031-3038). Ebenso ist der Zusatz von Säure möglich (Bioorg. Med. Chem., 2000, 8, 1415-22).

Verbindungen der allgemeinen Formel (II) mit R³ ≠ Wasserstoff können hergestellt werden nach folgendem Schema 2 :

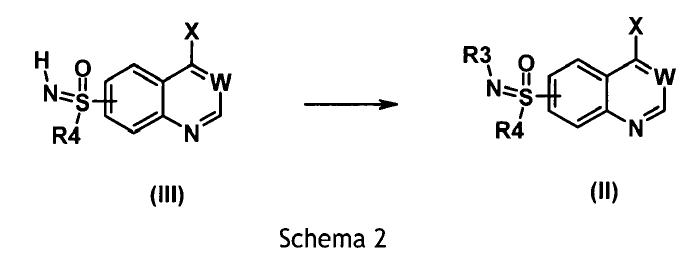

Ausgehend von Verbindungen der allgemeinen Formel (III) kann ein geeigneter Rest R³ durch Umsetzung eines entsprechend reaktiven Vorläufers in Anwesenheit z.B. einer Base und / oder einem Katalysator in einem eventuell nötigen Lösungsmittels in einem Temperaturbereich zwischen -78°C und +200°C eingeführt werden. Als geeigneter reaktiver Vorläufer kann ein Alkylhalogenid wie Methyljodid, Ethyljodid, Ethylbromid oder ein höheres Homolog aus der Reihe dienen. Als geeignete Base kann verwendet werden z.B. ein Amin wie Triethylamin, Pyridin, di-(iso)-Propylethylamin oder auch eine Base wie Natriumhydrid, Kaliumhydrid, Lithiumdiisopropylamin, Buthyllithium oder auch Salze wie Kaliumcarbonat, Cesiumcarbonat, Natriumcarbonat und ähnliche für den Fachmann gebräuchliche Basen. Ebenso kann ein Mono- wie auch ein Dialkylsulfat oder ein auf andere Weise und für den Fachmann bekannter, aktivierter Alkylrest verwendet werden. Solche Reaktionen sind beispielsweise beschrieben in (a) S. Gaillard, C. Papamicael, G. Dupas, F. Marsais, V. Levancher, Tetrahedron 2005, 61, 8138; (b) C. Bolm, H. Villar, Synthesis 2005, 9, 1421.

Ebenfalls können als aktivierte Reste z.B. Carbonyl oder Heterocarbonyl-Analoga verwendet werden wie beispielsweise Ameisensäurechlorid, Essigsäurechlorid, Essigsäureanhydrid, Methylsulfonsäurechlorid, Ethylisocyanat, Phenylisocyanat, Phenyliso-thiocyanat, Chlorameisensäuremethylester. Solche Reaktionen sind beispielsweise beschrieben in (a) M. Reggelin, H. Weinberger, V. Spohr, Adv. Synth. Catal. 2004, 346, 11, 1295, (b) T. Siu, A. Yudin, Org. Lett. 2002, 4, 1839, (c) V. J. Bauer, J. Org. Chem. 1966, 31, 3440 (d) A.C. Barnes et al., J. Med. Chem. 1979, 22, 418.
Verbindungen des Typs (III) können auch durch Verwendung von aktivierten aromatischen oder heteroaromatischen Ringsystemen in Gegenwart von geeigneten Übergangsmetallkatalysatoren zu Verbindungen mit der allgemeinen Formel (II) umgesetzt werden. Die Einführung dieser aromatischen und heteroaromatischen Reste über den Sulfoximid-Stickstoff kann durch Palladium-, Nickel- oder Kupfer-katalysierte Kreuzkupplungsreaktionen erreicht werden. Als aktivierter Aryl und Heteroaryl Rest kann beispielsweise ein Halogenid wie lodbenzol, Brombenzol, lodpyridin oder ähnliche als R3 definierte, mit einem Halogen versehene Reste verwendet werden. Ebenso können auf diese Weise Alkenylhalogenide oder Alkinylhalogenide umgesetzt werden. Solche Reaktionen sind beispielsweise beschrieben in (a) C. Bolm, J.P. Hildebrandt, Tetrahedron Lett. 1998, 39, 5731-5734; b) M. Harmata, N. Parvi, Angew. Chemie 1999, 38, 2577-2579; c) C. Bolm, J.P. Hildebrandt, J. Org. Chem. 2000, 65, 169; d) C. Bolm, J.P. Hildebrand, J. Rudolph, Synthesis 2000, 911-913; e) C. Bolm, M. Verrucci, O. Simic, P.G. Cozzi, G. Raabe, H. Okamura, Chem. Commun. 2003, 22, 2826-2827; f) G.Y. Cho, P. Rémy, J. Jansson, C. Moessner, C. Bolm, Org. Lett. 2004, 6, 3293-3296.)

Verbindungen der allgemeinen Formel (III) können hergestellt werden nach folgendem Schema 3:

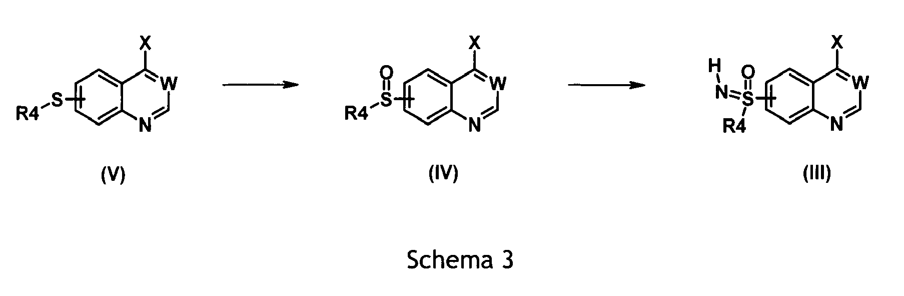

Schema 3 zeigt mögliche Herstellungsweisen der Sulfoximid-Bausteine. Thioether des Typs (V) können z.B. durch Oxidation mit Wasserstoffperoxid (Synthesis 2004, 227-232) in Sulfoxide des Typs (IV) überführt werden. Sulfoxide des Typs (III) lassen sich dann z.B. durch Umsetzung mit Natriumazid in Oleum in die Sulfoximide (IV) überführen (M. Reggelin, C. Zur, Synthesis, 2000, 1, 1). Die Reaktion kann in organischen Lösungsmitteln, wie z.B. Chloroform durchgeführt werden. Alternative Darstellungsweisen von Sulfoximiden aus Sulfoxiden oder Sulfiden sind ebenfalls beschrieben, z.B. unter Verwendung folgender Reagenzien
a) TsN₃ ((a) R. Tanaka, K. Yamabe, J. Chem. Soc. Chem. Commun. 1983, 329; (b) H. Kwart, A. A. Kahn, J. Am. Chem. Soc. 1967, 89, 1959))
b) N-Tosylimino-phenyl-iodinan und kat. Mengen Kupfer(I)triflat (J. F. K. Müller, P. Vogt, Tetrahedron Lett. 1998, 39, 4805)
c) Boc-azid und kat. Mengen Eisen(II)chloride (T. Bach, C. Korber, Tetrahedron Lett. 1998, 39, 5015)
d) o-Mesitylenesulfonylhydroxylamin (MSH) (C. R. Johnson, R. A. Kirchhoff, H. G. Corkins, J. Org. Chem. 1974, 39, 2458).
e) [N-(2-(Trimethylsilyl)ethanesulfonyl)imino]phenyliodinan (Phl=NSes) (S. Cren, T. C. Kinahan, C. L. Skinner and H. Tye, Tetrahedron Lett. 2002, 43, 2749).
f) Trifluoroacetamid, lodobenzoldiacetat, Magnesiumoxid und [Rh₂(OAc)₄] (H. Okamura and C. Bolm, Org. Lett. 2004, 6, 1305).
g) N-Bromsuccinimid oder lod und Cyanamin in Gegenwart einer Base wie Kalium-Tert-Butoxyd, gefolgt von einer Oxidation mit meta-Chlorperbenzoesäre (m-CPBA); O. G. Mancheno, O. Bistri, C. Bolm, Organic Letters 2007, 9, 3809-11.

Die Thioether des Typs (V) lassen sich herstellen analog z.B. (a) J. Med. Chem., 1994, 37, 2106; (b) Bioorg. Med. Chem. Lett., 2005, 15, 1015.

In Bezug auf Struktur und Konfiguration haben Sulfoximide im Allgemeinen eine hohe Stabilität (C. Bolm, J. P. Hildebrand, J. Org. Chem. 2000, 65, 169). Diese Eigenschaften der funktionellen Gruppe erlauben häufig drastische Bedingungen für nachfolgenden Reaktionen. Enantiomerenreine Sulfoximide werden auch als Auxiliare in der diastereoselectiven Synthese eingesetzt ((a) S. G. Pyne, Sulfur Reports 1992, 12, 57; (b) C. R. Johnson, Aldrichchimica Acta 1985, 18, 3). Die Darstellung enantiomerenreiner Sulfoximide ist ebenfalls beschrieben, z.B. durch Racematspaltung mit enantiomerenreiner Campher-10-sulfonsäure ((a) C. R. Johnson, C. W. Schroeck, J. Am. Chem. Soc. 1973, 95, 7418; (b) C. S. Shiner, A. H. Berks, J. Org. Chem. 1988, 53, 5543). Eine weitere Methode zur Herstellung optisch aktiver Sulfoximide ist die stereoselektive Iminierung optisch aktiver Sulfoxide unter Verwendung von MSH ((a) C. Bolm, P. Müller, K. Harms, Acta Chem. Scand. 1996, 50, 305; (b) Y. Tamura, J. Minamikawa, K. Sumoto, S. Fujii, M. Ikeda, J. Org. Chem. 1973, 38, 1239) oder Trifluoroacetamid, lodobenzoldiacetat, Magnesiumoxid und [Rh₂(OAc)₄] (H. Okamura and C. Bolm, Org. Lett. 2004, 6, 1305).

Ein weiterer Gegenstand der Erfindung betrifft Chinolin- und Chinazolin-Verbindungen der allgemeinen Formel (II), die als Zwischenstufen der Synthese dienen : 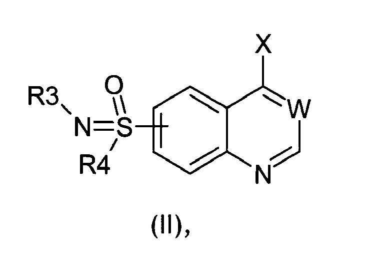 in der :
- W: gleich CH oder N ist;
- X: eine Halogen, eine OS(=O)₂R⁹-Gruppe, eine OP(=O)(OR⁹)₂-Gruppe oder eine (NR⁹R¹⁰R¹¹)⁺.Z⁻-Gruppe ist, wobei R⁹, R¹⁰ und R¹¹ unabhängig voneinander eine C₁-C₆-Alkyl-Gruppe oder C₆-C₁₂-Aryl-Gruppe ist, und Z⁻ ein Anion wie ein Halogenid ist ;
- R³: ausgewählt ist aus der Gruppe umfassend Wasserstoff, -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -Aryl, -Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -Aryl oder -Heteroaryl unsubstituiert oder einfach oder mehrfach unabhängig voneinander mit Hydroxy, Halogen, Nitro, Cyano, Phenyl, C(=O)R⁵, C(=O)NR⁵R⁶, C(=O)OR⁵, -NR⁵R⁶, Alkyl, -SR⁵ oder -OR⁵ substituiert ist,
- R⁴: ausgewählt ist aus der Gruppe umfassend -C₁-C₆-Atkyl, -C₂-C₆-Alkenyl, -C₂-C₆-Alkinyl, -C₃-C₁₀-Cycloalkyl, -C₃-C₁₂-Heterocycloalkyl, -Aryl, -Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -Aryl oder -Heteroaryl unsubstituiert oder einfach oder mehrfach unabhängig voneinander mit Hydroxy, Halogen, Nitro, Cyano, Phenyl, C(=O)R⁵, C(=O)OR⁵, C(=O)NR⁵R⁶, -NR⁵R⁶, Alkyl, -SR⁵ oder -OR⁵ substituiert ist,
- R⁵, R⁶: gleich oder verschieden und unabhängig voneinander ausgewählt sind aus der Gruppe, umfassend Wasserstoff, -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -Aryl und -Heteroaryl, wobei -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -Aryl oder -Heteroaryl unsubstituiert sind oder einfach oder mehrfach unabhängig voneinander mit Hydroxy, Halogen, Cyano, Nitro, -OR⁷, -NR⁷R⁸, - C(O)NR⁷R⁸, -C(O)OR⁷ oder -C₁-C₆-Alkyl, wobei -C₁-C₆-Alkyl unsubstituiert ist oder einfach oder mehrfach unabhängig voneinander mit Halogen, Hydroxy, Cyano, -NR⁷R⁸, -OR⁷ oder Phenyl; oder R⁵ und R⁶ optional miteinander eine Brücke aus 3-10 Methyleneinheiten bilden, wobei bis zu zwei Methyleneinheiten mit 0, S oder NR⁴ optional ersetzt sein können;
- R⁷, R⁸: gleich oder verschieden und unabhängig voneinander ausgewählt sind aus der Gruppe, umfassend Wasserstoff, -C₁-C₄-Alkyl, -Aryl und -Heteroaryl, wobei Alkyl, Aryl, Heteroaryl unsubstituiert ist oder einfach oder mehrfach unabhängig voneinander mit Halogen oder Alkoxy, oder R⁷ und R⁸ optional miteinander eine Brücke aus 3-10 Methyleneinheiten bilden, wobei bis zu zwei Methyleneinheiten mit 0, S oder -NR⁴ optional ersetzt sein können;
- R⁹, R¹⁰, R¹¹: gleich oder verschieden und unabhängig voneinander ausgewählt sind aus der Gruppe, umfassend -C₁-C₄-Alkyl, -Aryl und -Heteroaryl, wobei Alkyl, Aryl, Heteroaryl unsubstituiert ist oder einfach oder mehrfach unabhängig voneinander mit Halogen oder Alkoxy, oder R⁷ und R⁸ optional miteinander eine Brücke aus 3-10 Methyleneinheiten bilden, wobei bis zu zwei Methyleneinheiten mit 0, S oder -NR⁴ optional ersetzt sein können;

### EXPERIMENTELLER TEIL:

### Abkz.:

- δ =: chemische Verschiebung (ppm) in Relation zu Tetramethylsilan,
- s =: singulett, d = dublett, t = triplett, q = quartett, sep. = septett, m = multiplett, dm = dublett eines multipletts, bs = breites singulett
- THF =: Tetrahydrofuran
- L =: Liter
- g =: Gramm
- DMSO: = Dimethylsulfoxid
- RT =: Raumtemperatur
- min.: = Minuten

### Zwischenprodukt a):

**4-Chlor-6-methanesulfinyl-chinolin**

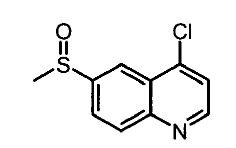

Eine Lösung von 62.2 g Natriumperjodat in 1.2 L Wasser wurde bei RT zu einer Lösung von 30.5 g 4-Chlor-6-methylsulfanyl-chinolin (Bioorg. Med. Chem. Lett. 15, (2005), pp. 1015-8) in 1.9 L THF getropft. Nach 60 h wurden 82.5 g Natriumsulfit zugesetzt und weitere 2 Stunden gerührt. Es wurde so viel Essigester zugegeben, bis sich zwei Phasen deutlich voneinander trennten. Die Lösung wurde filtriert und die Phasen anschließend getrennt. Die organische Phase wurde mit Natriumsulfat getrocknet und am Vakuum eingeengt. Das verblieben Öl wurde durch Säulenchromatographie (Kieselgel, Hexan / Essigester / 2-Propanol) aufgereinigt. Es wurden 25.5 g der Titelverbindung erhalten.

¹H-NMR (DMSO-d6): δ = 2.88 (s, 3H); 7.9 (d, 1H); 8.1 (dd, 1H); 8.27 (d, 1H); 8.51 (d, 1 H); 8.95 (d, 1 H);

### Zwischenprodukt b):

### S-(4-Chlor-chinolin-6-yl)-S-methylsulfoximid

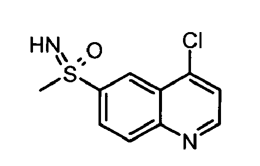

29.9 ml Oleum (20%) wurden zu einer Lösung von 4-Chlor-6-methanesulfinyl-chinolin in 116 ml Chloroform gegeben und die Innentemperatur auf ca. 45°C reguliert. Dazu wurden 8 g Natriumazid portionsweise über 90 Minuten zugegeben. Nach ca. 3 Stunden wurde der Ansatz auf Eiswasser gegossen und mit Natriumhydroxid vorsichtig auf pH = 11 eingestellt. Die organische Phase wurde getrennt und die wässrige Phase noch 3 Mal mit Dichlormethan extrahiert. Die vereinigten organischen Phasen wurden mit Natriumsulfat getrocknet und am Vakuum eingeengt. Das verbliebene Öl wurde durch Säulenchromatographie (Kieselgel, Hexan / Essigester / 2-Propanol) aufgereinigt. Es wurden 8.08 g der Titelverbindung erhalten.

¹H-NMR (DMSO-d6): δ = 3.43 (s, 3H); 7.95 (d, 1H); 8.33 (d, 2H); 8.78 (dd, 1H); 9.02 (d, 1H);

### Zwischenprodukt c):

### S-(4-chlor-chinolin-6-yl)-S-methyl-N-(allyloxycarbonyl)-sulfoximid

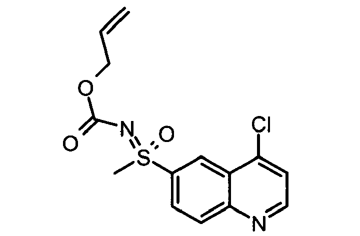

Eine Lösung von 3.7 g 4-Chlor-6-(S-methylsulfonimidoyl)-chinolin und 1.9 ml Chlorameisensäureallylester in 95 ml Pyridin wurde 15 Sunden bei RT gerührt.

Der Ansatz wurde auf 100 ml Eiswasser gegossen, 10 min. gerührt und mit 150 ml Toluol versetzt. Die organische Phase wurde getrennt und die wässrige Phase mit 150 ml Toluol extrahiert. Die vereinigten organischen Phasen wurden 1x mit 100ml halbgesättigter NaHCO₃-Lösung und 1x mit 100ml gesättigter NaCl-Lösung geschüttelt, über Natriumsulfat getrocknet und im Vakuum eingeengt. Das verblieben Öl wurde durch Säulenchromatographie (Kieselgel, Dichlormethan / Essigester / Methanol) aufgereinigt. Es wurden 4.1 g der Titelverbindung erhalten.

¹H-NMR (DMSO-d6): δ = 3.64 (s, 3H); 4.40 (dm, 2H); 5.11 (dm, 1H); 5.19 (dm,1H); 5.81 (ddt, 1H); 8.00 (d, 1H); 8.32 (dd, 1H); 8.38 (d, 1H); 8.78 (d, 1H); 9.07 (d, 1 H);

### Zwischenprodukt d):

### S-(4-Chlor-chinolin-6-yl)-S,N-dimethylsulfoximid

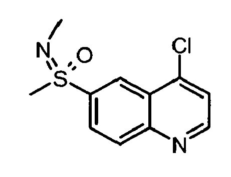

950 mg S-(4-Chlor-chinolin-6-yl)-S-methylsulfoximid wurden zu einer Suspension von 362 mg Natriumhydrid in 41 ml Dimethoxyethan bei RT gegeben. Dazu wurden 0.76 ml Methyljodid gegeben und bei RT für 72 Stunden gerührt. Der Ansatz wurde vollständig im Vakuum eingeengt und in Dichlormethan suspendiert. Die Suspension wurde filtriert und die organische Phase im Vakuum vollständig eingeengt. Der erhaltene Feststoff wurde durch Chromatographie (Kieselgel, Hexan/ Dichlormethan/ Methanol) gereinigt. Man erhielt 685 mg der Titelverbindung.

¹H-NMR (CDCl₃): δ = 2.71 (s, 3H); 3.20 (s, 3H); 7.65 (d, 1 H); 8.16 (dd, 1 H); 8.32 (d, 1 H); 8.89 (d, 1 H); 8.95 (d, 1 H);

### Zwischenprodukt e):

### N-[(4-Chlorchinolin-6-yl)(methyl)oxido- λ⁴-sulfanyliden]acetamid

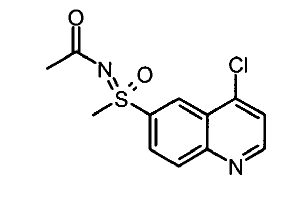

Eine Lösung von 200 mg (0.83 mmol) 4-Chlor-6-(S-methylsulfonimidoyl)-chinolin in 1 ml Pyridin wurde bei 0°C mit 78 mg (1 mmol) Acetylchlorid versetzt und über Nacht unter Rühren auf RT erwärmt. Anschließend wurden weitere 78 mg (1 mmol) Acetylchlorid zugegeben und noch 2 h bei RT gerührt. Der Ansatz wurde über präp. HPLC gereinigt. Es wurden 49 mg (20%) der Titelverbindung erhalten.
MS (ESpos): 283.3 [M+H]⁺.

### Zwischenprodukt f):

### Methyl-[(4-chlorchinolin-6-yl)(methyl)oxido-λ⁴-sulfanyliden]carbamat

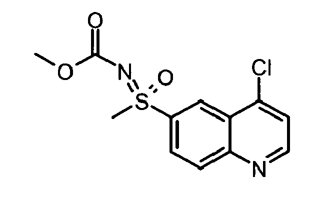

Eine Lösung von 200 mg (0.83 mmol) 4-Chlor-6-(S-methylsulfonimidoyl)-chinolin in 1 ml Pyridin wurde bei 0°C mit 94 mg (1 mmol) Chlorameisensäuremethylester versetzt und über Nacht unter Rühren auf RT erwärmt. Anschließend wurden weitere 94 mg (1 mmol) Chlorameisensäuremethylester zugegeben und noch 2 h bei RT gerührt. Der Ansatz wurde über präp. HPLC gereinigt. Es wurden 49 mg (20%) der Titelverbindung erhalten.
MS (ESpos): 299.0 [M+H]⁺.

### Zwischenprodukt g):

### 1-[(4-Chlorchinolin-6-yl)(methyl)oxido- λ⁴-sulfanyliden]-3-ethylharnstoff

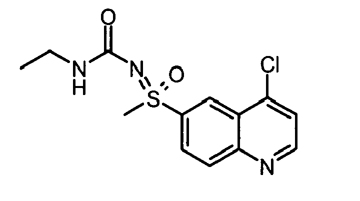

Eine Lösung von 200 mg (0.83 mmol) 4-Chlor-6-(S-methylsulfonimidoyl)-chinolin und 213 mg (3 mmol) Ethylisocyanat in 10 ml THF wurde 3 Tage bei 60°C gerührt. Der Ansatz wurde unter vermindertem Druck eingeengt und der Rückstand über präp. HPLC gereinigt. Es wurden 206 mg (80%) der Titelverbindung erhalten.
MS (ESpos): 312.3 [M+H]⁺.

### Beispiel 1:

### S-[4-(3-Hydroxyphenyl-1-yl)-aminochinolin-6-yl]-S-methylsulfoximid

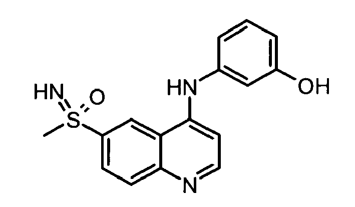

Eine Lösung von 65 mg S-(4-chlor-chinolin-6-yl)-S-methyl-N-(allyloxycarbonyl)-sulfoximid und 33 mg 3-Amino-phenol in 3 ml Acetonitril wurden für 8 Stunden bei 90°C gerührt. Das Lösungsmittel wurde vollständig im Vakuum entfernt und der Rückstand in 1 ml THF suspendiert. Dazu wurden 0.044 ml Morpholin und 23 mg Tetrakistriphenylphosphin Palladium zugegeben. Nach 22 Stunden wurde der Ansatz vollständig im Vakuum eingeengt und der Rückstand durch Flashchromatographie (Kieselgel, Dichlormethan / Methanol) gereinigt. Man erhielt 83 mg der Titelverbindung als Morpholin Salz.
¹H-NMR (DMSO-d6): δ = 3.01-3.12 (m, 4H); 3.20 (s, 3H); 3.73-3.84 (m, 4H); 6.61 (dd, 1H); 6.76-6.86 (m, 2H); 7.02 (d, 1H); 7.20 (t, 1H); 7.99 (d, 1H); 8.12 (dd, 1 H); 8.54 (d, 1 H); 9.09 (d, 1 H);

### Auf analoge Weise wie Bsp. 1 wurden hergestellt:

### Beispiel 2:

### S-[4-(3-Hydroxy-6-methyl-phenyl-1-yl)-aminochinolin-6-yl]-S-methylsulfoximid

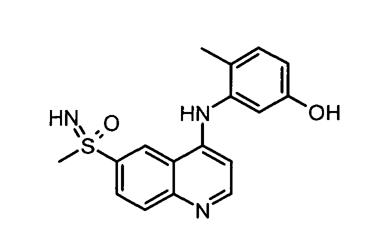

¹H-NMR (DMSO-d6): δ = 2.07 (s, 3H); 2.95-3.05 (m, 4H); 3.21 (s, 3H); 3.70-3.79 (m, 4H); 6.05 (d, 1 H); 6.73 (dd, 1 H); 6.81 (d, 1 H); 7.03 (d, 1 H); 7.94 (d, 1 H); 8.10 (dd, 1H); 8.39 (d, 1H); 9.13 (d, 1H);

### Beispiel 3:

### S-[4-(3-Hydroxy-5-methoxy-phenyl-1-yl)-aminochinolin-6-yl]-S-methylsulfoximid

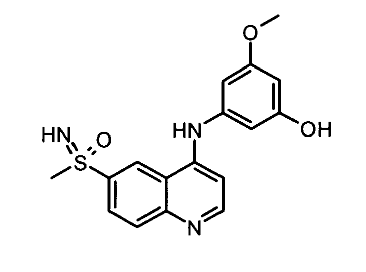

¹H-NMR (DMSO-d6): δ = 2.93-3.02 (m, 4H); 3.20 (s, 3H); 3.68-3.76 (m, 7H); 6.18 (t, 1H); 6.41 (t, 2H); 6.46 (t, 1H); 7.10 (d, 1H); 7.99 (d, 1H); 8.12 (dd, 1 H); 8.56 (d, 1 H); 9.06 (d, 1 H);

### Beispiel 4:

### S-[4-(6-Chlor-3-hydroxy-phenyl-1-yl)-aminochinolin-6-yl]-S-methylsulfoximid

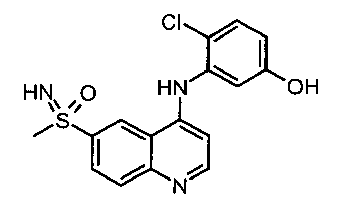

Eine Lösung von 100 mg (0.42 mmol) 4-Chlor-6-(S-methylsulfonimidoyl)-chinolin und 72 mg (0.5 mmol) 3-Amino-4-chlorphenol in 5 ml Isopropanol wurde mit 0.5 ml 4N HCl in Dioxan versetzt und über Nacht bei 80°C gerührt. Nach Abkühlen auf RT wurde mit Acetonitril verdünnt und der ausgefallene Niederschlag abfiltriert. Der Niederschlag wurde über präp. HPLC und Kieselgel chromatographiert. Es wurden 75 mg (52%) der Titelverbindung erhalten.
¹H-NMR (400 MHz, D6-DMSO): δ = 3.21 (s, 3H), 6.27 (bs, 1 H), 6.72-6.88 (m, 2H), 7.40 (d, 1 H), 7.98 (bs, 1 H), 8.13 (d, 1 H), 8.5 (bs, 1 H), 9.05 (s, 1 H), 9.41 (bs, 1 H), 9.9 (s, 1 H). MS (ESpos): 348.1 [M+H]⁺.

### Beispiel 5:

### S-[4-(3-Hydroxy-5-methoxy-phenyl-1-yl)-hydroxychinolin-6-yl]-S-methylsulfoximid

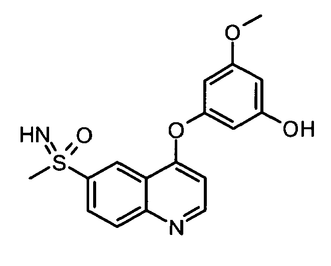

Eine Lösung von 100 mg (0.42 mmol) 4-Chlor-6-(S-methylsulfonimidoyl)-chinolin und 64 mg (0.46 mmol) 3,5-Dihydroxyanisol in 2.1 ml Acetonitril wurde mit 57 mg (0.42 mmol) Kaliumcarbonat versetzt und über Nacht unter Rückfluss gerührt. Nach Abkühlen auf RT wurde mit Ethylacetat verdünnt und 3 mal mit ges. Natriumhydrogencarbonat-Lösung sowie ges. NatriumchloridLösung gewaschen. Die org. Phase wurde über Natriumsulfat getrocknet und unter vermindertem Druck eingeengt. Der Rückstand wurde über präp. HPLC gereinigt. Es wurden 22 mg (15%) der Titelverbindung erhalten.
¹H-NMR (400 MHz, D6-DMSO): δ = 3.25 (s, 3H), 3.73 (s, 3H), 6.29 (t, 1 H), 6.35 (t, 1 H), 6.38 (t, 1 H), 6.87 (d, 1 H), 8.22 (d, 1 H), 8.28 (d, 1 H), 8.85-8.88 (m, 2H), 9.95 (s, 1 H). MS (ESpos): 345.1 [M+H]⁺.

### Beispiel 6:

### N,S-Dimethyl-S-[4-(3-hydroxyphenyl-1-yl)-aminochinolin-6-yl]-sulfoximid

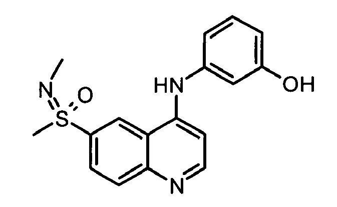

Eine Lösung von 51 mg S-(4-Chlor-chinolin-6-yl)-S,N-dimethylsulfoximid und 44 mg 3-Amino-phenol in 3 ml Acetonitril wurde für 22 Stunden bei 110°C gerührt. Das Lösungsmittel wurde vollständig im Vakuum entfernt. Der Rückstand wurde durch präparative HPLC getrennt: Säule: X-Bridge RP C18 30 x 100 5µM; Detektion: DAD TAC 200-400 nM; Fluss Rate: 50 ml/min; Laufmittel: A = 0.1% Ameisensäure in H₂O, B = Acetonitril; 10 min. Gradient. Man erhielt 48 mg der Titelverbindung.

¹H-NMR (DMSO-d6): δ = 2.51 (s, 3H); 3.23 (s, 3H); 6.58 (d, 1 H); 6.76-6.84 (m, 2H); 7.03 (d, 1H); 7.22 (t, 1H); 7.96-8.06 (m, 2H); 8.56 (d, 1H); 8.59 (s, 1H); 9.37 (bs, 1 H); 9.57 (bs, 1 H);

### Beispiel 7:

### N,S-Dimethyl-S-[4-(3-hydroxy-6-methyl-phenyl-1-yl)-aminochinolin-6-yl]-sulfoximid

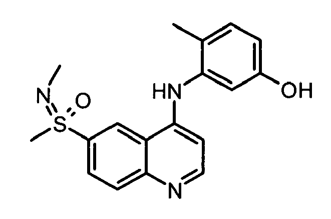

140.5 mg (0.552 mmol) S-(4-Chlor-chinolin-6-yl)-S,N-dimethylsulfoximid wurden unter Argonatmosphäre in 3 ml Acetonitril bei RT vorgelegt. Man gab 81.5 mg (0.662 mmol) 3-Amino-4-methylphenol und 138 µl einer 4 molaren Lösung von Chlorwasserstoff in 1,4-Dioxan hinzu und rührte 2 Std. bei 80°C. Man kühlte auf RT ab, stellte mit einnormaler Natronlauge einen basischen pH-Wert ein und extrahierte mit Ethylacetat. Die organische Phase wurde einmal mit gesättigter Natriumchloridlösung gewaschen. Man trocknete über Magnesiumsulfat und engte ein. Das Rohprodukt wurde durch Säulenchromatographie an Kieselgel (Laufmittel: Dichlormethan / Methanol = 10 : 1) gereinigt. Die produkthaltigen Fraktionen wurden vereinigt und eingeengt. Das erhaltene Produkt wurde im Hochvakuum getrocknet. Man erhielt 106 mg (56%) der Titelverbindung.

¹H-NMR (400 MHz, DMSO-d6): δ = 9.40 (br. s, 1H), 9.30 (s, 1H), 8.99 (s, 1H), 8.47 (d, 1H), 8.00 (q, 2H), 7.17 (d, 1H), 6.72-6.65 (m, 2H), 6.19 (d, 1H), 3.24 (s, 3H), 2.53 (s, 3H), 2.03 (s, 3H).

LC-MS: Rt= 0.60 min; MS (ESIpos): m/z = 342 [M+H]⁺.

### LC-MS-Methode:

Instrument: Micromass QuattroPremier mit Waters UPLC Acquity ; Säule: Thermo Hypersil GOLD 1,9µ 50x 1mm; Eluent A: 1l Wasser + 0,5ml 50%ige Ameisensäure , Eluent B: 1l Acetonitril + 0,5ml 50%ige Ameisensäure; Gradient: 0.0min 90%A 0.1min 90%A 1.5min 10%A 2.2min 10%A Ofen:50°C; Fluss: 0.33ml/min; UV-Detektion: 210 nm.

**Auf analoge Weise wie Bsp. 6 wurden hergestellt:**

### Beispiel 8:

### N,S-Dimethyl-S-[4-(3-hydroxy-5-methoxy-phenyl-1-yl)-aminochinolin-6-yl]-sulfoximid

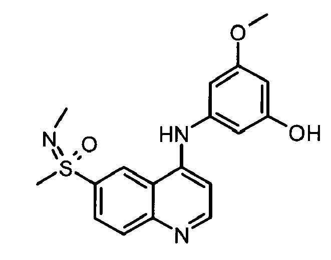

LC-MS: Säule: Acquity BEH C18 2,1X50 1,7 □M; Detektion: DAD TAC 200-400 nM; Fluss Rate: 0.8 ml/min; Laufmittel: A = 0.05% Ameisensäure in H₂O; B = Acetonitril; 2 min. Gradient (A=99 / B=1 -> A=1 / B=99) 1.6 min., (A = 1 / B = 99) 0.4 min; T = 60°C
MS (esi): M⁺+1 = 358 (M⁺+1 ber. = 358)
RT = 0.60 min.

### Beispiel 9:

### N-Acetyl-S-[4-(3-hydroxy-5-methoxy-phenyl-1-yl)-aminochinolin-6-yl]-S-methylsulfoximid

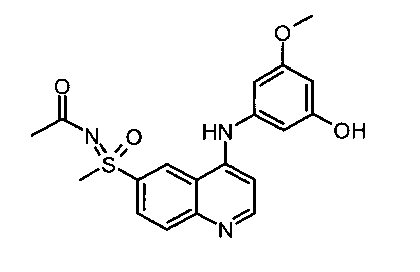

Eine Lösung von 37 mg (0.13 mmol) N-[(4-Chlorchinolin-6-yl)(methyl)oxido-λ⁴-sulfanyliden]acetamid und 24 mg (0.16 mmol) 3-Amino-5-methoxyphenol in 3 ml Isopropanol wurde mit 30 µl 4 N HCl in Dioxan versetzt und über Nacht bei 80°C gerührt. Der Lösung wurde eingeengt, in 1 ml Pyridin gelöst und bei 0°C mit 5 µl (0.07 mmol) Acetylchlorid versetzt. Der Ansatz wurde über Nacht bei RT gerührt, eingeengt und der Rückstand über Kieselgel chromatographiert. Es wurden 19 mg (35%) der Titelverbindung erhalten.
¹H-NMR (400 MHz, D6-DMSO): δ = 2.0 (s, 3H); 3.52 (s, 3H); 3.72 (s, 3H); 6.20 (t, 1 H); 6.4-6.43 (m, 2H); 7.10 (d, 1 H); 8.06 (d, 1 H); 8.11 (d, 1 H); 8.6 (d, 1 H); 9.07 (s, 1 H);9.51 (bs, 1 H); 9.65 (s, 1 H). MS (ESpos): 386.1 [M+H]⁺.

### Beispiel 10:

### N-Acetyl-S-[4-(3-Hydroxy-6-methyl-phenyl-1-yl)-aminochinolin-6-yl]-S-methylsulfoximid

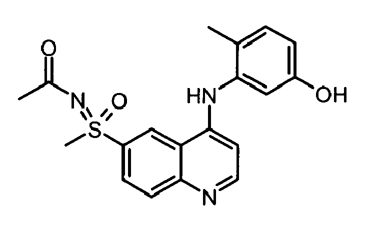

Eine Lösung von 43 mg (0.15 mmol) N-[(4-Chlorchinolin-6-yl)(methyl)oxido- λ⁴-sulfanyliden]acetamid und 22 mg (0.16 mmol) 3-Amino-4-methylphenol in 4 ml Acetonitril und 1 ml Methanol wurde 135 min in der Mikrowelle bei 150°C gerührt. Der Lösung wurde eingeengt und über Kieselgel chromatographiert. Es wurden 5 mg (9%) der Titelverbindung erhalten.
¹H-NMR (400 MHz, D6-DMSO): δ = 2.0 (s, 3H); 2.04 (s, 3H); 3.54 (s, 3H); 6.20 (d, 1 H); 6.66-6.73 (m, 2H); 7.18 (d, 1 H); 8.03 (d, 1 H); 8.09 (d, 1 H); 8.5 (d, 1 H); 9.1 (s, 1 H); 9.3 (bs, 1 H); 9.45 (s, 1 H). MS (ESpos): 370.0 [M+H]⁺.

### Beispiel 11:

### S-[4-(3-Hydroxy-6-methyl-phenyl-1-yl)-aminochinolin-6-yl]-N-methoxy-carbonyl-S-methylsulfoximid

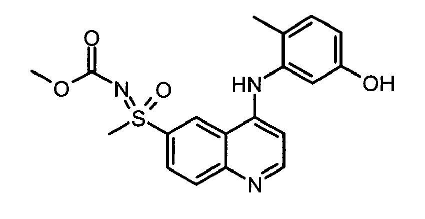

Eine Lösung von 34 mg (0.11 mmol) Methyl-[(4-chlorchinolin-6-yl)(methyl)oxido-λ⁴-sulfanyliden]carbamat und 17 mg (0.14 mmol) 3-Amino-4-methylphenol in 3 ml Isopropanol wurde mit 30 µl 4 N HCl in Dioxan versetzt und 6 h bei 80°C gerührt. Die Lösung wurde eingeengt und über präp. HPLC und Kieselgel chromatographiert. Es wurden 21 mg (48%) der Titelverbindung erhalten.
¹H-NMR (400 MHz, D6-DMSO): δ = 2.08 (s, 3H); 3.51 (s, 3H); 3.67 (s, 3H); 6.38 (d, 1 H); 6.78 (d, 1 H); 6.86 (dd, 1 H); 7.28 (d, 1 H); 8.25 (d, 1 H); 8.45 (d, 1 H); 8.56 (d, 1 H); 9.5 (s, 1 H); 9.8 (bs, 1 H). MS (ESpos): 386.1 [M+H]⁺.

### Beispiel 12:

### S-[4-(3-Hydroxy-5-methoxy-phenyl-1-yl)-aminochinolin-6-yl]-N-methoxy-carbonyl-S-methylsulfoximid

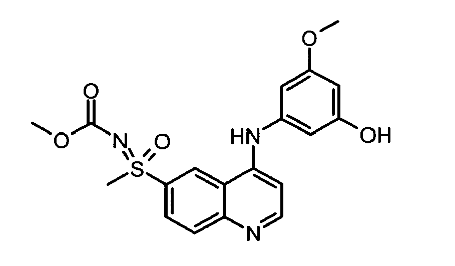

Eine Lösung von 34 mg (0.11 mmol) Methyl-[(4-chlorchinolin-6-yl)(methyl)oxido-λ⁴-sulfanyliden]carbamat und 19 mg (0.14 mmol) 3-Amino-5-methoxyphenol in 3 ml Isopropanol wurde mit 30 µl 4 N HCl in Dioxan versetzt und 6 h bei 80°C gerührt. Die Lösung wurde eingeengt und über präp. HPLC chromatographiert. Es wurden 47 mg (95%) der Titelverbindung erhalten. ¹H-NMR (400 MHz, D6-DMSO): δ = 3.48 (s, 3H); 3.66 (s, 3H); 3.73 (s, 3H); 6.38 (d, 1 H); 6.5 (d, 2H); 7.02 (d, 1 H); 8.24 (d, 1 H); 8.36 (d, 1 H); 8.60 (d, 1 H); 9.41 (s, 1 H); 9.98 (bs, 1 H). MS (ESpos): 402.1 [M+H]⁺.

### Beispiel 13:

### N-(Ethylamino)carbonyl-S-[4-(3-Hydroxy-5-methoxy-phenyl-1-yl)-aminochinolin-6-yl]-S-methylsulfoximid

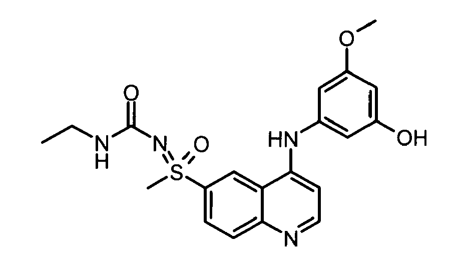

Eine Lösung von 100 mg (0.32 mmol) 1-[(4-Chlorchinolin-6-yl)(methyl)oxido-λ⁴-sulfanyliden]-3-ethylharnstoff und 60 mg (0.39 mmol) 3-Amino-5-methoxyphenol in 3 ml Isopropanol wurde mit 30 µl 4 N HCl in Dioxan versetzt und über Nacht bei 80°C gerührt. Die Lösung wurde eingeengt und über präp. HPLC und Kieselgel chromatographiert. Es wurden 88 mg (65%) der Titelverbindung erhalten.
¹H-NMR (400 MHz, D6-DMSO): δ = 0.96 (t, 3H); 2.93 (m, 2H); 3.50 (s, 3H); 3.75 (s, 3H); 6.43 (t, 1 H); 6.5 (d, 2H); 6.98 (d, 1 H); 8.22 (d, 1 H); 8.39 (d, 1 H); 8.59 (d, 1 H); 9.41 (s, 1 H); 10.03 (bs, 1 H), 11.4 (s, 1 H). MS (ESpos): 415.2 [M+H]⁺.

### Beispiel 14:

### N-(Ethylamino)carbonyl-S-[4-(3-Hydroxy-6-methyl-phenyl-1-yl)-aminochinolin-6-yl]-S-methylsulfoximid

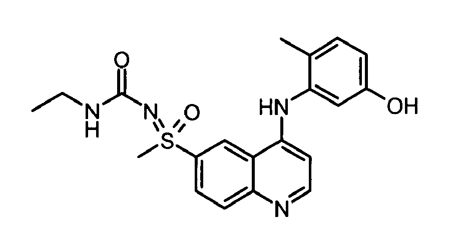

Eine Lösung von 100 mg (0.32 mmol) 1-[(4-Chlorchinolin-6-yl)(methyl)oxido-λ⁴-sulfanyliden]-3-ethylharnstoff und 47 mg (0.39 mmol) 3-Amino-4-methylphenol in 5 ml Isopropanol wurde mit 50 µl 4 N HCl in Dioxan versetzt und 6 h bei 80°C gerührt. Der erhaltene Niederschlag wurde abgesaugt und im Hochvakuum getrocknet Es wurden 87 mg (62%) der Titelverbindung als Hydrochlorid-Salz erhalten.
¹H-NMR (400 MHz, D6-DMSO): δ = 0.96 (t, 3H); 2.09 (s, 3H); 2.93 (m, 2H); 3.50 (s, 3H); 3.58 (s, 3H); 6.37 (t, 1 H); 6.77 (d, 1 H); 6.85 (dd, 1 H); 7.08 (t, 1 H); 7.28 (d, 1H; 8.20 (d, 1H); 8.39 (d, 1H); 8.55 (d, 1H); 9.42 (s, 1H); 9.75 (bs, 1H); 11.35 (bs, 1H), 14.6 (s, 1H). MS (ESpos): 399.2 [M+H]⁺.

### Beispiel 15: S-[4-(3-Hydroxy-5-methoxy-phenyl-1-yl)-aminochinazolin-6-yl]-S-methylsulfoximid

### Zwischenprodukt 15a) :

### N-(3-Methoxy-5-hydroxy-phenyl)-6-methylsulfanyl-chinazolin-4-amin

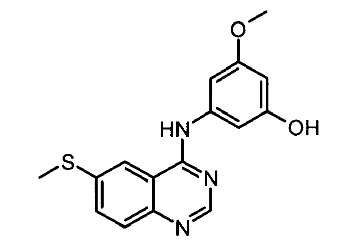

Eine Lösung von 210 mg 4-Chlor-6-methylsulfanylchinazolin (J. Med. Chem. 26, (1983), 420 ff.; J. Med. Chem. 37, (1994), 2106 ff.) und 278 mg 5-Amino-3-methoxyphenol in 15 ml Acetonitril wurden für 48 Stunden bei 110°C gerührt. Nach Abkühlung wurde mit Essigester verdünnt und der entstandene Niederschlag abfiltriert. Der Niederschlag wurde mit Essigester gewaschen und im Vakuum getrocknet. Man erhielt 333 mg der Titelverbindung, die als Hydrochlorid vorlag.

¹H-NMR (DMSO-d6): δ = 2.70 (s, 3H); 6.35 (t, 1H); 6.74-6.80 (m, 2H); 7.88 (d, 1 H); 7.98 (dd, 1 H); 8.56 (d, 1 H); 8.87 (s, 1 H); 9.84 (s, 1 H); 11.48 (s, 1 H);

### Zwischenprodukt 15b) : N-(3-Methoxy-5-hydroxy-phenyl)-6-methylsulfinylchinazolin-4-amin

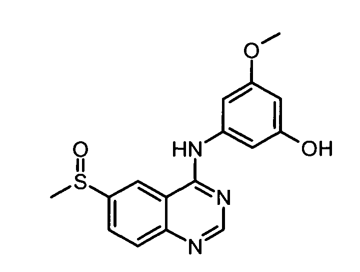

Eine Lösung von 455 mg Natriumperjodat in 9 mL Wasser wurde bei RT zu einer Lösung von 333 mg N-(3-Methoxy-5-hydroxy-phenyl)-6-methylsulfanyl-chinazolin-4-amin in 18 mL THF getropft. Nach 40 h wurden weitere 445 mg Natriumperjodat in 9 mL Wasser zugegeben und noch 24 h gerührt. Es wurde so viel Essigester zugegeben, bis sich zwei Phasen deutlich voneinander trennten. Die organische Phase wurde getrennt und die wässrige Phase wurde 2 Mal mit einer 9:1 Mischung aus Dichlormethan / Iso-Propanol extrahiert. Die vereinigten organischen Phasen wurden mit Natriumsulfat getrocknet und am Vakuum eingeengt.

¹H-NMR (DMSO-d6): δ = 2.87 (s, 3H); 3.73 (s, 3H); 6.16 (t, 1H); 6.94 (t, 1H); 7.07 (t, 1 H); 7.94 (d, 1 H); 8.11 (dd, 1 H); 8.67 (s, 1 H); 8.89 (d, 1 H); 9.49 (s, 1 H); 9.93 (s, 1 H);

### Endprodukt : Beispiel 15: S-[4-(3-Hydroxy-5-methoxy-phenyl-1-yl)-aminochinazolin-6-yl]-S-methylsulfoximid

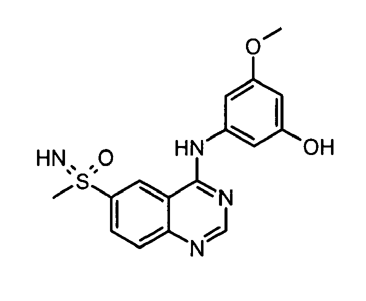

12 µl Oleum (20%) wurden zu einer Lösung von 14.43 mg N-(3-Methoxy-5-hydroxy-phenyl)-6-methylsulfinyl-chinazolin-4-amin in 200 µl Chloroform gegeben und die Innentemperatur auf ca. 50°C reguliert. Dazu wurden 3.19 mg Natriumazid zugegeben. Nach ca. 3 Stunden wurde der Ansatz auf Eiswasser gegeben und mit Natronlauge vorsichtig auf pH = 11 eingestellt. Die organische Phase wurde getrennt und die wässrige Phase noch 3 Mal mit Dichlormethan extrahiert. Die vereinigten organischen Phasen wurden mit Natriumsulfat getrocknet und am Vakuum eingeengt. Man erhielt die Titelverbindung nach Säulenchromatographie (Kieselgel, Hexan / Essigester / 2-Propanol).

¹H-NMR (DMSO-d6): δ = 3.20 (s, 3H); 4.42 (s, 1H); 6.17 (t, 1H); 6.93 (t, 1H); 7.04 (t, 1 H); 7.91 (d, 1 H); 8.28 (dd, 1 H); 8.68 (s, 1 H); 9.22 (d, 1 H); 9.56 (s, 1 H); 10.13 (s, 1 H);

### Auf analoge Weise wurden hergestellt:

### Beispiel 16:

**S-[4-(3-Hydroxy-phenyl-1-yl)-aminochinazolin-6-yl]-S-**methylsulfoximid

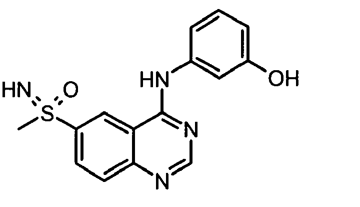

Ausgehend von 4-Chlor-6-methylsulfanylchinazolin und 3-Aminophenol wurde die Titelverbindung über die Zwischenstufen N-(3-Hydroxy-phenyl)-6-methylsulfanyl-chinazolin-4-aminN-(3-Hydroxy-phenyl)-6-methylsulfinyl-chinazolin-4-amin hergestellt.
¹H-NMR (DMSO-d6): δ = 3.18 (s, 3H); 4.39 (s, 1H); 6.49-6.60 (m, 1H); 7.09-7.22 (m, 2H); 7.30 (s, 1 H); 7.87 (d, 1 H); 8.25 (dd, 1 H); 8.60 (s, 1 H); 9.17 (s, 1 H); 10.17 (bs, 1H);

### Beispiel 17:

**S-[4-(3-Hydroxy-6-methyl-phenyl-1-yl)-aminochinazolin-6-yl]-S-methylsulfoximid**

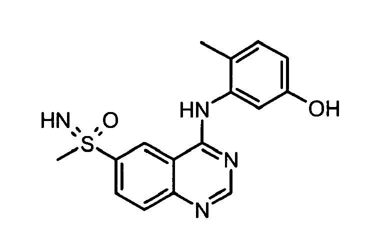

Ausgehend von 4-Chlor-6-methylsulfanylchinazolin und 3-Amino-4-methylphenol wurde die Titelverbindung über die Zwischenstufen N-(3-Hydroxy-6-methyl-phenyl)-6-methylsulfanyl-chinazolin-4-amin N-(3-Hydroxy-6-methyl-phenyl)-6-methylsulfinyl-chinazolin-4-amin hergestellt.

¹H-NMR (DMSO-d6): δ = 3.19 (s, 3H); 4.37 (s, 1 H); 6.63 (d, 1 H); 6.69 (d, 1 H); 7.07 (d, 1 H); 7.86 (d, 1 H); 8.24 (d, 1 H); 8.46 (s, 1 H); 9.10 (s, 1 H); 9.31 (s, 1H); 10.14 (s, 1H);

### Beispiel 18:

**S-[4-(3-Hydroxy-6-methyl-phenyl-1-yl)-aminochinazolin-6-yl]-S-methylsulfoximid**

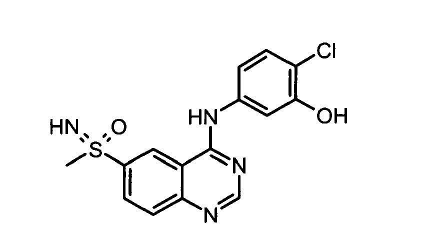

Ausgehend von 4-Chlor-6-methylsulfanylchinazolin und 5-Amino-2-chlorphenol wurde die Titelverbindung über die Zwischenstufen N-(4-Chlor-3-hydroxy-phenyl)-6-methylsulfanyl-chinazolin-4-amin N-(4-Chlor-3-hydroxyphenyl)-6-methylsulfinyl-chinazolin-4-amin hergestellt.

¹H-NMR (DMSO-d6): δ = 3.19 [s, 3H); 4,43 (s, 1H); 7.27 (dd, 1H); 7.34 (d, 1H); 7.63 (d, 1H); 7.93 (d, 1H); 8.30 (dd, 1H); 8.69 (s, 1H); 9.21 (d, 1H); 10.26 (s, 2H);

### BIOLOGISCHE TESTS DER VERBINDUNGEN DER BETREFFENDEN ERFINDUNG

### TESTSYSTEM FÜR EphB4

### HTRF Test:

Eine Mischung aus 20 ng/ml rekombinanter EphB4 kinase (ProQinase GmbH, Freiburg, Germany), 2.67 µg/ml polyGluAlaTyr, 2 µM ATP, 25 mM HEPES (pH 7.3), 5 mM MgCl₂, 1 mM MnCl₂, 2 mM DTT, 0.1 mM NaVO₄, 1% (v/v) Glycerin, 0.02% NP40, EDTA-freie Protease Inhibitoren (Complete Fa. Roche, 1 Tablette in 50 ml) wird bei 20 °C 10 min lang inkubiert. Testsubstanzen werden in 100% DMSO gelöst und in 0.017-fachem Volumen vor dem Start der Reaktion vorgelegt. 60 Minuten nach Zugabe von 1.7-fachem Volumen einer Lösung 50mM Hepes pH 7.0, 0.2 % BSA, 0.14 µg/ml PT66-Europium, 3.84 µg/ml SA-XL665, 75 mM EDTA wird der Ansatz in einem Discovery HTRF-Messgerät der Firma Perkin-Elmer vermessen.

### Kaskaden Test:

Die Wirkstärke der erfindungsgemäßen Verbindungen wird in einem *in vitro-*Hemmtest ermittelt. Die EphB4-Kinase vermittelte Phosphorylierung eines geeigneten Peptidsubstrates geht einher mit einem Verbrauch von ATP und einer entsprechenden Zunahme von ADP. ADP wird in einer gekoppelten enzymatischen Reaktion unter NADH-Verbrauch nachgewiesen (Pyruvatkinase/Lactatdehydrogenase Reaktion; PK/LDH). Die intrinsische Fluoreszenz von NADH dient als sensitives Auslesesignal des Tests. Der NADH Verbrauch (Abnahme der Fluoreszenzintensität) ist direkt proportional zur EphB4- Enzymaktivität. Die Wirkkonzentration einer Testverbindung, bei der die Hälfte des Enzyms inhibiert ist (50% Signalintensität des Fluoreszenzlichtes), wird als IC₅₀-Wert angegeben.
In einer 384 Loch-Mikrotiterplatte werden in einem Testvolumen von insgesamt 42 µl Testpuffer (50 mM MOPS pH 7.0, 5 mM MgCl₂, 0.3 mM ZnCl₂, 2.5 mM EDTA, 1 mM DTE, 0.01% w/v BSA, 0.006 % v/v Igepal, 0.001 % w/v Histon), Enzym (5 nM EphB4, Fa. Millipore, Heidelberg; 0.5 U/ml PK/LDH, Fa. Roche, Mannheim) und Substrat (0.1 mg/ml polyGluAlaTyr; 100 µM ATP, 50 µM NADH, 1 mM PEP) bei An- und Abwesenheit der Testsubstanz (Verdünnungen in DMSO) zwei Stunden bei 32°C inkubiert. Die Fluoreszenzlichtintensität der Testansätze wird gemessen (Tecan Safire, Ex/Em. 340/465 nm, TECAN, Crailsheim). Die IC₅₀-Werte werden durch eine Auftragung der Fluoreszenzlichtintensität gegenüber der Wirkstoffkonzentration ermittelt.

### Inhibition der EphB4-Autophosphorylierung in CHO-EphB4-Zellen:

Die Bindung des Liganden EphrinB2 als Dimer (EphrinB2-Fc) an den EphB4 Rezeptor führt zur Aktivierung und Autophosphorylierung des Rezeptors. Diese Autophosphorylierung kann durch EphB4-Kinase Inhibitoren gehemmt werden. Die phosphorylierte Form des Rezeptors, der eine c-Myc Sequenz enthält, kann in einem Sandwich-Elisa nachgewiesen werden. EphB4-Kinase Inhibitoren reduzieren dosisabhängig die Menge des phosphorylierten EphB4 Rezeptors. 80,000 stabil transfizierte CHO-EphB4 Zellen/well werden in eine 96-well Zellkulturplatte ausgesät und über Nacht in DMEM/HAMS-F12/10% FCS bei 37°C und 5% CO2 kultiviert (Sturz A. et al. 2004, Biochem. Biophys. Acta 313, 80-88, modifiziert). Danach werden die Zellen eimal mit PBS gewaschen, in serumfreiem Medium für 10 min mit Substanzen vorbehandelt und anschließend mit 0.5µg/ml (bis 1 µg/ml) ephrinB2-Fc für 60 min bei 4°C inkubiert. Nach der Behandlung werden die Zellen lysiert in 100µl/well LysePuffer (50mM HEPES pH 7.2, 150mM NaCl, 1 mM MgCl2, 10mM Na4P207, 100mM NaF, 10% Glycerin (v/v), 1.5% Triton X-100, 2mM Orthovanadate (pH 7.4), mit "complete Proteaseinhibitors" (Roche) und Phosphatase Inhibitor Cocktail II (Sigma)) und 30 min inkubiert. Autophosphorylierter EphB4-Rezeptor wird in einem Sandwich-ELISA mit anti c-Myc- und anti-PhosphoTyrosin-HRP- Antikörpern detektiert. Dazu werden 96-well Platten (Lumitrac 600, Greiner) mit 100µl/well anti-c-myc Antikörper (5µg/ml; Roche) über Nacht bei 4°C inkubiert, anschließend 3 Stunden bei RT mit 250µl/well 5% Prionex (Calbiochem) geblockt und danach bei 4°C mit 100µl Zelllysat über Nacht inkubiert. Die ELISA-Platten werden danach zweimal mit PBS gewaschen und mit 100µl/well anti-Phospho-Tyr-HRP (1:10000 in Lysepuffer) über Nacht bei 4°C inkubiert. Dann werden die Platten dreimal mit PBS gewaschen, mit 100µl/well BM Chemilumineszenz ELISA-Substrat POD (Roche) versetzt und die Lumineszenz in einem Luminometer gemessen (LumiCount, Packard oder Lumibox, Bayer).

### BIOLOGIE

Verbindungen der betreffenden Erfindung besitzen überraschenderweise inhibitorische Aktivität gegen EphB4 mit IC50-Werten kleiner als 10 µM in mindestens eine der oben genannten Test Systeme.

## Patentansprüche

1. Chinolin- bzw. Chinazolinderivate mit der allgemeinen Formel (I) : 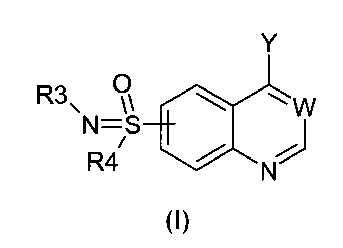 in der :
W gleich CH oder N ist ;
Y gleich NR¹R² oder OR¹ ist ;
R¹ und R² gleich oder verschieden und einfach oder mehrfach unabhängig voneinander ausgewählt sind aus der Gruppe, umfassend Wasserstoff, -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, -C₁-C₆-Alkyl-C₁-C₆-alkoxy, -C₁-C₆-Alkyl-C₁-C₆-alkoxy-C₁-C₆-alkoxy, -(CH₂)ₙ-C₆-C₁₂-Aryl, -(CH₂)ₙ-C₅-C₁₈-Heteroaryl,-(CH₂)ₙ-C₃-C₁₀-Cycloalkyl, -(CH₂)ₙ-C₃-C₁₂-Heterocycloalkyl,-Phenylen-(CH₂)p-R⁶, -(CH₂)ₚPO₃(R⁶)₂, -(CH₂)ₚ-NR⁴C(=S)R⁵, -(CH₂)ₚ-NR⁴S(=O)R⁵, -(CH₂)ₚ-NR⁴C(=O)NR⁵R⁶ -(CH₂)ₚ-NR⁴C(=O)OR⁵,-(CH₂)ₚ-NR⁴C(=NH)NR⁵R⁶, -(CH₂)ₚ-NR⁴C(=S)NR⁵R⁶, -(CH₂)ₚ-NR⁴S(=O)NR⁵R⁶, -(CH₂)ₚ-NR⁴S(=O)₂NR⁵R⁶, -(CH₂)ₚ-C(=O)R⁵, -(CH₂)ₚ-C(=S)R⁵, -(CH₂)ₚ-S(=O)R⁵, -(CH₂)ₚ-S(O)(NH)R⁵, -(CH₂)ₚ-S(O)₂R⁵,-(CH₂)ₚ-SO₂OR⁵, -(CH₂)ₚ-CO₂R⁵, -(CH₂)ₚ-CSNR⁵R⁶, -CHR⁵R⁶ und-(CH₂)ₚ-SR⁵ wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl oder -C₁-C₆-Alkoxy unsubstituiert sind oder einfach oder mehrfach unabhängig voneinander mit Hydroxy, Halogen, Nitro, Cyano, -NR⁵R⁶, -C(O)NR⁵R , -S(O)₂NR⁵R⁶, -NR⁵S(O)₂R⁶,-NR⁵C(O)R⁶, -SR⁵ , -R⁵, oder -OR⁵ substituiert sind, wobei das Kohlenstoffgerüst des -C₃-C₁₀-Cycloalkyls und des -C₁-C₁₀-Alkyls einfach oder mehrfach unabhängig voneinander Stickstoff-, Sauerstoff-, Schwefelatome, -NR⁴ oder C=0-Gruppen oder eine oder mehrere Doppelbindungen enthalten kann, oder R¹ und R² optional miteinander eine Brücke aus 3-10 Methyleneinheiten bilden, wobei bis zu zwei Methyleneinheiten mit 0, S oder -NR⁴ optional ersetzt werden, und wobei der Phenyl-Rest optional einfach oder mehrfach unabhängig voneinander mit Hydroxy, Halogen, Nitro, Cyano, Phenyl, -NR⁵R⁶, Alkyl oder -OR⁵ substituiert ist ;
worin wenn Y NR¹R² ist, und R² Wasserstoff ist, dann R¹ nicht Wasserstoff ist ;
und worin wenn Y OR¹ ist, dann R¹ nicht Wasserstoff ist ;
R³ ausgewählt ist aus der Gruppe umfassend Wasserstoff, -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)NR⁵R⁶, C(=O)OR⁵, -NR⁵R⁶, Alkyl, -SR⁵ oder -OR⁵ substituiert ist,
R⁴ ausgewählt ist aus der Gruppe umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)OR⁵, C(=O)NR⁵R⁶, -NR⁵R⁶, Alkyl, -SR⁵ oder -OR⁵ substituiert ist,
oder
R³ und R⁴ können, über das jeweilige Stickstoffatom und das jeweilige Schwefelatom, an das sie angeknüpft sind, einen Ring bilden, mit der Ringgröße von 5- bis zu 10- Ringatomen, wahlweise bestehend aus den Atomen Kohlenstoff, Stickstoff, Sauerstoff oder Schwefel ;
R⁵ und R⁶ gleich oder verschieden und unabhängig voneinander ausgewählt sind aus der Gruppe, umfassend Wasserstoff, -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -C₆-C₁₂-Aryl und -C₅-C₁₈-Heteroaryl, wobei -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -C₆-C₁₂-Aryl oder -C₅-C₁₈-Heteroaryl unsubstituiert sind oder einfach oder mehrfach unabhängig voneinander mit Hydroxy, Halogen, Cyano, Nitro, -OR⁷, -NR⁷R⁸, -C(O)NR⁷R⁸, -C(O)OR⁷ oder -C₁-C₆-Alkyl, wobei -C₁-C₆-Alkyl unsubstituiert ist oder einfach oder mehrfach unabhängig voneinander mit Halogen, Hydroxy, Cyano, -NR⁷R⁸, -OR⁷ oder Phenyl; oder R⁵ und R⁶ optional miteinander eine Brücke aus 3-10 Methyleneinheiten bilden, wobei bis zu zwei Methyleneinheiten mit 0, S oder NR⁴ optional ersetzt sein können;
R⁷, R⁸ gleich oder verschieden und unabhängig voneinander ausgewählt sind aus der Gruppe, umfassend Wasserstoff, -C₁-C₄-Alkyl, - C₅-C₁₈-Aryl und - C₅-C₁₈-Heteroaryl, wobei -C₁-C₄-Alkyl, -C₆-C₁₂-Aryl, -C₅-C₁₈-Heteroaryl unsubstituiert ist oder einfach oder mehrfach unabhängig voneinander mit Halogen oder Alkoxy, oder R⁷ und R⁸ optional miteinander eine Brücke aus 3-10 Methyleneinheiten bilden, wobei bis zu zwei Methyleneinheiten mit 0, S oder -NR⁴ optional ersetzt sein können;
n = 1, 2, 3, 4, 5, oder 6,
p = 0, 1, 2, 3, 4, 5, oder 6, sowie
deren N-Oxide, Solvate, Hydrate, Stereoisomere, Diastereomere, Enantiomere und Salze.

2. Chinolin- bzw. Chinazolinderivate der allgemeinen Formel (I) nach dem Anspruch 1, worin :
W gleich CH oder N ist ;
Y gleich NR¹R² oder OR¹ ist ;
R¹ einfach oder mehrfach unabhängig voneinander ausgewählt ist aus der Gruppe, umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, -C₁-C₆-Alkyl-C₁-C₆-alkoxy, -C₁-C₆-Alkyl-C₁-C₆-alkoxy-C₁-C₆-alkoxy, -(CH₂)ₙ-C₆-C₁₂-Aryl, -(CH₂)ₙ-C₅-C₁₈-Heteroaryl, - (CH₂)ₙ-C₃-C₁₀-Cycloalkyl, -(CH₂)ₙ-C₃-C₁₂-Heterocycloalkyl, -Phenylen-(CH₂)ₚ-R⁶, -(CH₂)ₚPO₃(R⁶)₂, -(CH₂)ₚ-NR⁴C(=S)R⁵, -(CH₂)ₚ-NR⁴S(=O)R⁵, -(CH₂)ₚ-NR⁴C(=O)NR⁵R⁶, -(CH₂)ₚ-NR⁴C(=O)OR⁵, -(CH₂)ₚ-NR⁴C(=NH)NR⁵R⁶, -(CH₂)ₚ-NR⁴C(=S)NR⁵R⁶, -(CH₂)ₚ-NR⁴S(=O)NR⁵R⁶, -(CH₂)ₚ-NR⁴S(=O)₂NR⁵R⁶, -(CH₂)ₚ-C(=O)R⁵, -(CH₂)ₚ-C(=S)R⁵, -(CH₂)ₚ-S(=O)R⁵, -(CH₂)ₚ-S(O)(NH)R⁵, -(CH₂)ₚ-S(O)₂R⁵, -(CH₂)ₚ-SO₂OR⁵, -(CH₂)ₚ-CO₂R⁵, -(CH₂)ₚ-CSNR⁵R⁶, -CHR⁵R⁶ und -(CH₂)ₚ-SR⁵ ; wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl oder -C₁-C₆-Alkoxy unsubstituiert ist oder einfach oder mehrfach unabhängig voneinander mit Hydroxy, Halogen, Nitro, Cyano, -NR⁵R⁶, -C(O)NR⁵R⁶, -S(O)₂NR⁵R⁶, -NR⁵S(O)₂R⁶, -NR⁵C(O)R⁶, -SR⁵, -R⁵, oder -OR⁵ substituiert ist, wobei das Kohlenstoffgerüst des -C₃-C₁₀-Cycloalkyls und des -C₁-C₁₀-Alkyls einfach oder mehrfach unabhängig voneinander Stickstoff-, Sauerstoff-, Schwefelatome, -NR⁴ oder C=0-Gruppen oder eine oder mehrere Doppelbindungen enthalten kann,
R² einfach oder mehrfach unabhängig voneinander ausgewählt ist aus der Gruppe, umfassend Wasserstoff, -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -(CH₂)ₙ-C₃-C₁₀-Cycloalkyl, -(CH₂)ₙ-C₃-C₁₂-Heterocycloalkyl, und -Phenylen-(CH₂)ₚ-R⁶, wobei -C₁-C₆-Alkyl, - C₂-C₆-Alkenyl, -C₂-C₆-Alkinyl, -C₃-C₁₀-Cycloalkyl, -C₃-C₁₂-Heterocycloalkyl, -C₆-C₁₂-Aryl oder -C₅-C₁₈-Heteroaryl unsubstituiert ist, wobei das Kohlenstoffgerüst des -C₃-C₁₀-Cycloalkyls und des -C₁-C₁₀-Alkyls einfach oder mehrfach unabhängig voneinander Stickstoff-, Sauerstoff-, Schwefelatome, -NR⁴ oder C=O-Gruppen oder eine oder mehrere Doppelbindungen enthalten kann ;
oder:
R¹ und R² optional miteinander eine Brücke aus 3-10 Methyleneinheiten bilden, wobei bis zu zwei Methyleneinheiten mit 0, S oder -NR⁴ optional ersetzt werden, und wobei der Phenyl-Rest optional einfach oder mehrfach unabhängig voneinander mit Hydroxy, Halogen, Nitro, Cyano, Phenyl, -NR⁵R⁶, Alkyl oder -OR⁵ substituiert ist ;
R³ ausgewählt ist aus der Gruppe umfassend Wasserstoff, -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)NR⁵R⁶, C(=O)OR⁵, -NR⁵R⁶, Alkyl, -SR⁵ oder -OR⁵ substituiert ist,
R⁴ ausgewählt ist aus der Gruppe umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)OR⁵, C(=O)NR⁵R⁶, -NR⁵R⁶, Alkyl, -SR⁵ oder -OR⁵ substituiert ist,
R⁵ und R⁶ gleich oder verschieden und unabhängig voneinander ausgewählt sind aus der Gruppe, umfassend Wasserstoff, -C₁-C₁₀-Alkyl, -C₂-C₁₀-Alkenyl, -C₂-C₁₀-Alkinyl, -C₁-C₆-Alkoxy, -C₃-C₁₀-Cycloalkyl,-C₃-C₁₂-Heterocycloatkyt, -C₆-C₁₂-Aryl und -C₅-C₁₈-Heteroaryl, wobei -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -C₆-C₁₂-Aryl oder -C₅-C₁₈-Heteroaryl unsubstituiert sind oder einfach oder mehrfach unabhängig voneinander mit Hydroxy, Halogen, Cyano, Nitro, -OR⁷, -NR⁷R⁸, -C(O)NR⁷R⁸, -C(O)OR⁷ oder -C₁-C₆-Alkyl, wobei -C₁-C₆-Alkyl unsubstituiert ist oder einfach oder mehrfach unabhängig voneinander mit Halogen, Hydroxy, Cyano, -NR⁷R⁸, -OR⁷ oder Phenyl; oder R⁵ und R⁶ optional miteinander eine Brücke aus 3-10 Methyleneinheiten bilden, wobei bis zu zwei Methyleneinheiten mit 0, S oder NR⁴ optional ersetzt sein können;
R⁷, R⁸ gleich oder verschieden und unabhängig voneinander ausgewählt sind aus der Gruppe, umfassend Wasserstoff, -C₁-C₄-Alkyl, - C₅-C₁₈-Aryl und - C₅-C₁₈-Heteroaryl, wobei -C₁-C₄-Alkyl, -C₆-C₁₂-Aryl, -C₅-C₁₈-Heteroaryl unsubstituiert ist oder einfach oder mehrfach unabhängig voneinander mit Halogen oder Alkoxy, oder R⁷ und R⁸ optional miteinander eine Brücke aus 3-10 Methyleneinheiten bilden, wobei bis zu zwei Methyleneinheiten mit 0, S oder -NR⁸ optional ersetzt sein können;
n = 1, 2, 3, 4, 5, oder 6,
p = 0, 1, 2, 3, 4, 5, oder 6, sowie
deren N-Oxide, Solvate, Hydrate, Stereoisomere, Diastereomere, Enantiomere und Salze.

3. Chinolin- bzw. Chinazolinderivate der allgemeinen Formel (I) nach dem Anspruch 1 oder 2, worin :
W gleich CH oder N ist ;
Y gleich NR¹R² oder OR¹ ist ;
R¹ einfach oder mehrfach unabhängig voneinander ausgewählt ist aus der Gruppe, umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, -C₁-C₆-Alkyl-C₁-C₆-alkoxy, -C₁-C₆-Alkyl-C₁-C₆-alkoxy-C₁-C₆-alkoxy, -(CH₂)ₙ-C₆-C₁₂-Aryl, -(CH₂)ₙ-C₅-C₁₈-Heteroaryl, -(CH₂)ₙ-C₃-C₁₀-Cycloalkyl, -(CH₂)ₙ-C₃-C₁₂-Heterocycloalkyl, -Phenylen-(CH₂)ₚ-R⁶, -(CH₂)ₚPO₃(R⁶)₂, -(CH₂)ₚ-NR⁴C(=S)R⁵, -(CH₂)ₚ-NR⁴S(=O)R⁵, -(CH₂)ₚ-NR⁴C(=O)NR⁵R⁶, -(CH₂)ₚ-NR⁴C(=O)OR⁵, -(CH₂)ₚ-NR⁴C(=NH)NR⁵R⁶, -(CH₂)ₚ-NR⁴C(=S)NR⁵R⁶, -(CH₂)ₚ-NR⁴S(=O)NR⁵R⁶, -(CH₂)ₚ-NR⁴S(=O)₂NR⁵R⁶, -(CH₂)ₚ-C(=O)R⁵, -(CH₂)ₚ-C(=S)R⁵, -(CH₂)ₚ-S(=O)R⁵, -(CH₂)ₚ-S(O)(NH)R⁵, -(CH₂)ₚ-S(O)₂R⁵, -(CH₂)ₚ-SO₂OR⁵, -(CH₂)ₚ-CO₂R⁵, -(CH₂)ₚ-CSNR⁵R⁶, -CHR⁵R⁶ und -(CH₂)ₚ-SR⁵ ; wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl oder -C₁-C₆-Alkoxy unsubstituiert ist oder einfach oder mehrfach unabhängig voneinander mit Hydroxy, Halogen, Nitro, Cyano, -NR⁵R⁶, -C(O)NR⁵R⁶, -S(O)₂NR⁵R⁶, -NR⁵S(O)₂R⁶, -NR⁵C(O)R⁶, -SR⁵, -R⁵, oder -OR⁵ substituiert ist, wobei das Kohlenstoffgerüst des -C₃-C₁₀-Cycloalkyls und des -C₁-C₁₀-Alkyls einfach oder mehrfach unabhängig voneinander Stickstoff-, Sauerstoff-, Schwefelatome, -NR⁴ oder C=O-Gruppen oder eine oder mehrere Doppelbindungen enthalten kann,
R² einfach oder mehrfach unabhängig voneinander ausgewählt ist aus der Gruppe, umfassend Wasserstoff, oder -C₁-C₆-Alkyl ;
R³ ausgewählt ist aus der Gruppe umfassend Wasserstoff, -C₁-C₆-Alkyl, -C₂-C₆-Alkenyl, -C₂-C₆-Alkinyl, -C₃-C₁₀-Cycloatkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)NR⁵R⁶, C(=O)OR⁵, -NR⁵R⁶, Alkyl, -SR⁵ oder -OR⁵ substituiert ist,
R⁴ ausgewählt ist aus der Gruppe umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)OR⁵, C(=O)NR⁵R⁶, -NR⁵R⁶, Alkyl, -SR⁵ oder -OR⁵ substituiert ist,
R⁵ und R⁶ gleich oder verschieden und unabhängig voneinander ausgewählt sind aus der Gruppe, umfassend Wasserstoff, -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -C₆-C₁₂-Aryl und -C₅-C₁₈-Heteroaryl, wobei -C₁-C₁₀-Alkyl, -C₂-C₁₀-Alkenyl, -C₂-C₁₀-Alkinyl, -C₁-C₆-Alkoxy, -C₃-C₁₀-Cycloalkyl, -C₃-C₁₂-Heterocycloalkyl, -C₆-C₁₂-Aryl oder -C₅-C₁₈-Heteroaryl unsubstituiert sind oder einfach oder mehrfach unabhängig voneinander mit Hydroxy, Halogen, Cyano, Nitro, -OR⁷, -NR⁷R⁸, -C(O)NR⁷R⁸, -C(O)OR⁷ oder -C₁-C₆-Alkyl, wobei -C₁-C₆-Alkyl unsubstituiert ist oder einfach oder mehrfach unabhängig voneinander mit Halogen, Hydroxy, Cyano, -NR⁷R⁸, -OR⁷ oder Phenyl; oder R⁵ und R⁶ optional miteinander eine Brücke aus 3-10 Methyleneinheiten bilden, wobei bis zu zwei Methyleneinheiten mit 0, S oder NR⁴ optional ersetzt sein können;
R⁷, R⁸ gleich oder verschieden und unabhängig voneinander ausgewählt sind aus der Gruppe, umfassend Wasserstoff, -C₁-C₄-Alkyl, - C₅-C₁₈-Aryl und - C₅-C₁₈-Heteroaryl, wobei -Cₗ-C₄-Atkyl, -C₆-C₁₂-Aryl, -C₅-C₁₈-Heteroaryl unsubstituiert ist oder einfach oder mehrfach unabhängig voneinander mit Halogen oder Alkoxy, oder R⁷ und R⁸ optional miteinander eine Brücke aus 3-10 Methyleneinheiten bilden, wobei bis zu zwei Methyleneinheiten mit 0, S oder -NR⁴ optional ersetzt sein können;
n = 1, 2, 3, 4, 5, oder 6,
p = 0, 1, 2, 3, 4, 5, oder 6, sowie
deren N-Oxide, Solvate, Hydrate, Stereoisomere, Diastereomere, Enantiomere und Salze.

4. Chinolin- bzw. Chinazolinderivate der allgemeinen Formel (I) nach einem der Ansprüche 1, 2 oder 3, worin :
W gleich CH oder N ist ;
Y gleich NR¹R² oder OR¹ ist ;
R¹ einfach oder mehrfach unabhängig voneinander ausgewählt ist aus der Gruppe, umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, -C₁-C₆-Alkyl-C₁-C₆-alkoxy, -C₁-C₆-Alkyl-C₁-C₆-atkoxy-C₁-C₆-alkoxy, -(CH₂)ₙ-C₆-C₁₂-Aryl, -(CH₂)ₙ-C₅-C₁₈-Heteroaryl, -(CH₂)ₙ-C₃-C₁₀-Cycloalkyl, -(CH₂)ₙ-C₃-C₁₂-Heterocycloalkyl, -Phenylen-(CH₂)ₚ-R⁶, -(CH₂)ₚPO₃(R⁶)₂, -(CH₂)ₚ-NR⁴C(=S)R⁵, -(CH₂)ₚ-NR⁴S(=O)R⁵, -(CH₂)ₚ-NR⁴C(=O)NR⁵R⁶, -(CH₂)ₚ-NR⁴C(=O)OR⁵ -(CH₂)p-NR⁴C(=NH)NR⁵R⁶, -(CH₂)ₚ-NR⁴C(=S)NR⁵R⁶, -(CH₂)ₚ-NR⁴S(=O)NR⁵R⁶, -(CH₂)ₚ-NR⁴S(=O)₂NR⁵R⁶,-(CH₂)ₚ-C(=O)R⁵, -(CH₂)ₚ-C(=S)R⁵, -(CH₂)ₚ-S(=O)R⁵, -(CH₂)ₚ-S(O)(NH)R⁵, -(CH₂)ₚ-S(O)₂R⁵, -(CH₂)ₚ-SO₂OR⁵,-(CH₂)ₚ-CO₂R⁵, -(CH₂)ₚ-CSNR⁵R⁶, -CHR⁵R⁶ und -(CH₂)ₚ-SR⁵; wobei-C₁-C₆-Alkyl, -C₂-C₆-Alkenyl, -C₂-C₆-Alkinyl, -C₃-C₁₀-Cyctoatkyt, -C₃-C₁₂-Heterocycloalkyl, -C₆-C₁₂-Aryl, -C₅-C₁₈-Heteroaryl oder -C₁-C₆-Alkoxy unsubstituiert ist oder einfach oder mehrfach unabhängig voneinander mit Hydroxy, Halogen, Nitro, Cyano, -NR⁵R⁶,-C(O)NR⁵R⁶, -S(O)₂NR⁵R⁶, -NR⁵S(O)₂R⁶, -NR⁵C(O)R⁶, -SR⁵, -R⁵, oder -OR⁵ substituiert ist, wobei das Kohlenstoffgerüst des -C₃-C₁₀-Cycloalkyls und des -C₁-C₁₀-Alkyls einfach oder mehrfach unabhängig voneinander Stickstoff-, Sauerstoff-, Schwefelatome, -NR⁴ oder C=O-Gruppen oder eine oder mehrere Doppelbindungen enthalten kann,
R² einfach oder mehrfach unabhängig voneinander ausgewählt ist aus der Gruppe, umfassend Wasserstoff, oder -C₁-C₆-Alkyl ;
R³ ausgewählt ist aus der Gruppe umfassend Wasserstoff, -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, -C₅-C₁₈-Heteroaryl, C(=O)R⁵, C(=O)NR⁵R⁶ oder C(=O)OR⁵, wobei -C₁-C₆-Alkyl, -C₂-C₆-Atkenyt, -C₂-C₆-Alkinyl, -C₃-C₁₀-Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)NR⁵R⁶, C(=O)OR⁵, -NR⁵R⁶, Alkyl, -SR⁵ oder -OR⁵ substituiert ist,
R⁴ ausgewählt ist aus der Gruppe umfassend -C₁-C₆-Alkyl, -C₂-C₆-Alkenyl, -C₂-C₆-Alkinyl, -C₃-C₁₀-Cycloalkyl, -C₃-C₁₂-Heterocycloalkyl, -C₆-C₁₂-Aryl, oder -C₅-C₁₈-Heteroaryl, wobei -C₁-C₆-Alkyl, -C₂-C₆-Alkenyl, -C₂-C₆-Alkinyl, -C₃-C₁₀-Cycloalkyl, -C₃-C₁₂-Heterocycloalkyl , -C₆-C₁₂-Aryl oder -C₅-C₁₈-Heteroaryl unsubstituiert oder einfach oder mehrfach unabhängig voneinander mit Hydroxy, Halogen, Nitro, Cyano, Phenyl, C(=O)R⁵, C(=O)OR⁵, C(=O)NR⁵R⁶, -NR⁵R⁶, Alkyl, -SR⁵ oder -OR⁵ substituiert ist,
R⁵ und R⁶ gleich oder verschieden und unabhängig voneinander ausgewählt sind aus der Gruppe, umfassend Wasserstoff, -C₁-C₁₀-Alkyl, -C₂-C₁₀-Alkenyl, -C₂-C₁₀-Alkinyl, -C₁-C₆-Alkoxy, -C₃-C₁₀-Cycloalkyt, -C₃-C₁₂-Heterocycloalkyl, -C₆-C₁₂-Aryl und -C₅-C₁₈-Heteroaryl, wobei -C₁-C₁₀-Alkyl, -C₂-C₁₀-Alkenyl, -C₂-C₁₀-Alkinyl, -C₁-C₆-Alkoxy, -C₃-C₁₀-Cycloatkyl, -C₃-C₁₂-Heterocycloalkyl, -C₆-C₁₂-Aryl oder -C₅-C₁₈-Heteroaryl unsubstituiert sind oder einfach oder mehrfach unabhängig voneinander mit Hydroxy, Halogen, Cyano, Nitro, -OR⁷, -NR⁷R⁸, -C(O)NR⁷R⁸, -C(O)OR⁷ oder -C₁-C₆-Alkyl, wobei -C₁-C₆-Alkyl unsubstituiert ist oder einfach oder mehrfach unabhängig voneinander mit Halogen, Hydroxy, Cyano, -NR⁷R⁸, -OR⁷ oder Phenyl; oder R⁵ und R⁶ optional miteinander eine Brücke aus 3-10 Methyleneinheiten bilden, wobei bis zu zwei Methyleneinheiten mit 0, S oder NR⁴ optional ersetzt sein können;
R⁷, R⁸ gleich oder verschieden und unabhängig voneinander ausgewählt sind aus der Gruppe, umfassend Wasserstoff, -C₁-C₄-Alkyl, - C₅-C₁₈-Aryl und - C₅-C₁₈-Heteroaryl, wobei -C₁-C₄-Alkyl, -C₆-C₁₂-Aryl, -C₅-C₁₈-Heteroaryl unsubstituiert ist oder einfach oder mehrfach unabhängig voneinander mit Halogen oder Alkoxy, oder R⁷ und R⁸ optional miteinander eine Brücke aus 3-10 Methyleneinheiten bilden, wobei bis zu zwei Methyleneinheiten mit 0, S oder -NR⁴ optional ersetzt sein können;
n = 1, 2, 3, 4, 5, oder 6,
p = 0, 1, 2, 3, 4, 5, oder 6, sowie
deren N-Oxide, Sotvate, Hydrate, Stereoisomere, Diastereomere, Enantiomere und Salze.

5. Chinolin- bzw. Chinazolinderivate mit der allgemeinen Formel (I) nach einem der Ansprüche 1 bis 4, das ausgewählt ist aus :
S-[4-(3-Hydroxyphenyl-1-yl)-aminochinolin-6-yl]-S-methylsulfoximid ;S-[4-(3-Hydroxy-6-methyl-phenyl-1-yl)-aminochinolin-6-yl]-S-methylsulfoximid ;S-[4-(3-Hydroxy-5-methoxy-phenyl-1-yl)-aminochinolin-6-yl]-S-methylsulfoximid; S-[4-(6-Chlor-3-hydroxy-phenyl-1-yl)-aminochinolin-6-yl]-S-methylsulfoximid ; S-[4-(3-Hydroxy-5-methoxy-phenyl-1-yl)-hydroxychinolin-6-yl]-S-methylsulfoximid ;
N,S-Dimethyl-S-[4-(3-hydroxyphenyl-1-yl)-aminochinolin-6-yl]-sulfoximid ;N,S-Dimethyl-S-[4-(3-hydroxy-6-methyl-phenyl-1-yl)-aminochinolin-6-yl]-sulfoximid ;N,S-Dimethyl-S-[4-(3-hydroxy-5-methoxy-phenyl-1-yl)-aminochinolin-6-yl]-sulfoximid ;N-Acetyl-S-[4-(3-hydroxy-5-methoxy-phenyl-1-yl)-aminochinolin-6-yl]-S-methylsulfoximid ; N-Acetyl-S-[4-(3-Hydroxy-6-methyl-phenyl-1-yl)-aminochinolin-6-yl]-S-methylsulfoximid ;
S-[4-(3-Hydroxy-6-methyl-phenyl-1-yl)-aminochinolin-6-yl]-N-methoxycarbonyl-S-methylsulfoximid ;
S-[4-(3-Hydroxy-5-methoxy-phenyl-1-yl)-aminochinolin-6-yl]-N-methoxycarbonyl-S-methylsulfoximid ;
N-(Ethylamino)carbonyl-S-[4-(3-Hydroxy-5-methoxy-phenyl-1-yl)-aminochinolin-6-yl]-S-methylsulfoximid ;
N-(Ethylamino)carbonyl-S-[4-(3-Hydroxy-6-methyl-phenyl-1-yl)-aminochinolin-6-yl]-S-methylsulfoximid ;
S-[4-(3-Hydroxy-5-methoxy-phenyl-1-yl)-aminochinazolin-6-yl]-S-methylsulfoximid ;
S-[4-(3-Hydroxy-phenyl-1-yl)-aminochinazolin-6-yl]-S-methylsulfoximid ;
S-[4-(3-Hydroxy-6-methyl-phenyl-1-yl)-aminochinazolin-6-yl]-S-methylsulfoximid ; und
S-[4-(3-Hydroxy-6-methyl-phenyl-1-yl)-aminochinazolin-6-yl]-S-methylsulfoximid.

6. Verfahren zur Herstellung des Chinolin- bzw. Chinazolin-derivats nach einem der Ansprüche 1 bis 5, wobei eine Zwischenstufe der allgemeinen Formel (II) : 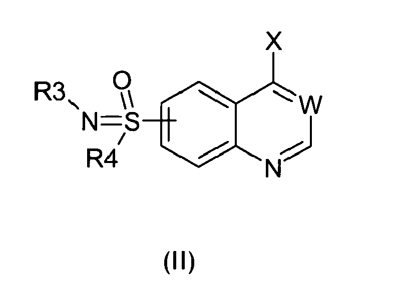 in der :
W, R³ und R⁴ wie in einem der Ansprüche 1 bis 4 definiert sind ;
X eine Halogen, eine -OS(=O)₂R⁹-Gruppe, eine -OS(=O)₂CₓF₂ₓ₊₁ Gruppe, eine - OP(=O)(OR⁹)₂-Gruppe oder eine -(NR⁹R¹⁰R¹¹)⁺.Z⁻-Gruppe ist ;
R⁹, R¹⁰, R¹¹ unabhängig voneinander eine C₁-C₆-Alkyl-Gruppe oder C₆-C₁₂-ArylGruppe ist ;
Z⁻ ein Anion wie ein Halogenid ist ; und
X = 1, 2, 3, 4, oder 5 ist ;
umgesetzt wird mit einem Reagenz der allgemeinen Formel (A) :
HY
(A),
in der Y wie in einem der Ansprüche 1 bis 4 definiert sind,
zu einer Verbindung der allgemeinen Formel (I) : 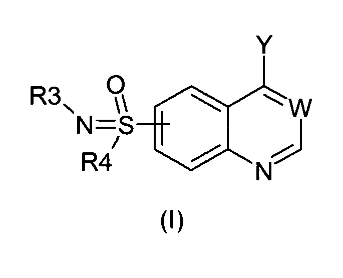 in der W, R³, R⁴ und Y wie in einem der Ansprüche 1 bis 4 definiert sind.

7. Verwendung des Chinolin- bzw. Chinazolin-derivats nach einem der Ansprüche 1 bis 5, oder wie gemäß Anspruch 6 herstellbaren, zur Herstellung eines Arzneimittels.

8. Verwendung des Chinolin- bzw. Chinazolin-derivats nach einem der Ansprüche 1 bis 5, oder wie gemäß Anspruch 6 herstellbaren, zur Herstellung eines Arzneimittels zur Behandlung von Erkrankungen, in denen die Angiogenese, Lymphangiogenese oder Vaskulogenese eine Rolle spielen, von Erkrankungen der Blutgefäße, von Erkrankungen, die durch eine Hyperproliferation von Körperzellen hervorgerufen werden, sowie von chronischen oder akuten neurodegenerativen Erkrankungen.

9. Verwendung des Chinolin- bzw. Chinazotin-derivats nach einem der Ansprüche 1 bis 5, oder wie gemäß Anspruch 6 herstellbaren, für diagnostische Zwecke *in vitro* oder *in vivo* zur Identifizierung von Rezeptoren in Geweben mittels Autoradiographie oder PET.

10. Verwendung des Chinolin- bzw. Chinazolin-derivats nach einem der Ansprüche 1 bis 5, oder wie gemäß Anspruch 6 herstellbaren, als Inhibitor der Eph-Rezeptor Kinasen.

11. Verwendung des Chinolin- bzw. Chinazolin-derivats nach einem der Ansprüche 1 bis 5, oder wie gemäß Anspruch 6 herstellbaren, in Form eines pharmazeutischen Präparates für die enterale, parenterale und orale Applikation.

12. Arzneimittel, die mindestens ein Chinolin- bzw. Chinazolin-derivats nach einem der Ansprüche 1 bis 5, oder wie gemäß Anspruch 6 herstellbaren, sowie geeignete Formulierungs- und Trägerstoffe enthalten.

13. Zwischenprodukt der allgemeinen Formel (II) : 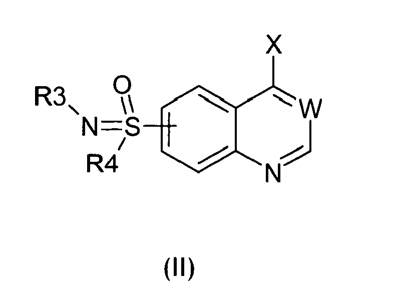 in der :
W, R³ und R⁴ wie in einem der Ansprüche 1 bis 4 definiert sind, und X eine Halogen, eine OS(=O)₂R⁹-Gruppe, eine OP(=O)(OR⁹)₂-Gruppe oder eine (NR⁹R¹⁰R¹¹)⁺.Z⁻-Gruppe ist, wobei R⁹, R¹⁰ und R¹¹ unabhängig voneinander eine C₁-C₆-Alkyl-Gruppe oder C₆-C₁₂-Aryl-Gruppe ist, und Z⁻ ein Anion wie ein Halogenid ist.

14. Verwendung eines der Zwischenprodukte der allgemeinen Formel (II) nach dem Ansprüche 13, zur Herstellung eines Chinolin- bzw. Chinazolin-derivats der allgemeinen Formel (I) nach einem der Ansprüche 1 bis 5.
